# EUROPEAN PATENT SPECIFICATION

(11) **EP 3 193 743 B1**
(45) Date of publication and mention of the grant of the patent: **27.11.2019**
(21) Application number: 15763021.1
(22) Date of filing: 15.09.2015
(51) Int. Cl.: A61B 17/12

(54) **AN EMBOLISATION DEVICE**
EMBOLISIERUNGSVORRICHTUNG
DISPOSITIF D'EMBOLISATION

(30) Priority: 15.09.2014 EP 14184807; 21.01.2015 EP 15151922; 03.07.2015 EP 15175292
(43) Date of publication of application: 26.07.2017
(73) Proprietor: Embo Medical Limited, Enniscorthy, County Wexford (IE)
(72) Inventor: MULLINS, Liam, County Westmeath (IE); FORDE, Colin, County Galway (IE); ALLEN, Wayne, County Galway (IE); SHERIDAN, William, County Cavan (IE); GILSON, Paul, County Galway (IE)
(74) Representative: Hoffmann Eitle
(86) International application number: PCT/EP2015/071097
(87) International publication number: WO 2016/041961

(56) References cited:
- EP-A1- 0 778 006
- WO-A1-2013/025493
- WO-A1-2014/140325
- DE-A1- 19 607 451
- US-A- 5 573 547
- US-A- 6 159 206
- US-A1- 2007 227 544

## Description

### Introduction

This invention relates to an embolisation device.

Migration of conventional embolisation coils occurs in 4-14% of transcatheter embolisations [1,2].

Non-target embolisation is an outcome of coils migration, the impact of which depends on the final location of the coils. In the venous system, the consequences can be catastrophic with literature indicating that coils can migrate into the renal vein, right atrium of the heart, lung (pulmonary artery). Percutaneous retrieval of the coils is technically very challenging and frequently cannot be attempted as the coils are often entrapped within the organs and tissue. Coil migration occurs for various reasons:
- Technical error: release of a coil or coil pack too distal or proximal to an adjoining larger vessel or plexus [3,4]
- High blood flow areas can cause the coil to migrate.
- Coil: vessel mismatch. The coils are undersized, hence will not injure the vessel wall, will not induce thrombosis, and are likely to migrate. Or the coils are oversized and will act like a guide-wire and pass further distally into the vessel [5,6].
- Vessel dilation: coil migration can occur due to a disparity in the size of coils and dilated vessels, which can change in their diameters depending on vessel hemodynamics [7].
- Coils impart a very low radial (anchor) force on the lumen, once a clot forms within the coil, blood flow can force it to migrate.

The profile of the embolisation device and delivery system is a critical success factor in successfully accessing target embolisation locations e.g. the iliac arteries are frequently tortuous in the presence of abdominal aortic aneurysms [5]. To combat this issue today, microcatheters are often employed in difficult or tortuous anatomy where use of standard catheters may induce spasm and lead to a failed embolisation procedure [5]. Additionally different stages in a procedure may require catheters with different mechanical properties e.g. accessing a visceral vessel, particularly in the presence of diseased or tortuous arteries, may require a catheter with a high degree of stiffness and torque control. In general, the lower the profile of the device and delivery system, the greater the accessibility of the device into tortuous and higher order vessels. A lower profile device reduces the diameter of catheter required for delivery and lowers the risks of access site infections, hematomas and lumen spasm.

Dependent on the clinical application of the device, variable anchor forces may be required to prevent migration of the prosthesis e.g. arterial and venous applications have variable blood flow rates and forces. This in turn, will lead to a compromise in terms of profile since in order to anchor the device stiffer, and consequently larger elements may be required. For example in the case of a bristle device larger diameter fibres may be required.

The technique generally used to embolise vessels today is to insert a metallic scaffold (coil, plug) into the target vessel, to cause a thrombus that adheres to the scaffold, relying on the thrombus to induce blood cessation and eventually occlude the vessel. In general, available embolisation technology does not interfere with or interact with blood flow densely enough across the vessel cross section to induce rapid, permanent vessel occlusion.

Using technology available today, the physician will often have to prolong a specific duration of time for the technology to induce occlusion. In one approach the physician inserts coils and then waits 20 minutes for the coils to expand and cause vessel occlusion [8].

The restoration of the lumen of a blood vessel following thrombotic occlusion by restoration of the channel or by the formation of new channels, is termed recanalisation. Recanalisation can occur due to, coil migration, fragmentation of the embolisation material, and formation of a new vessel lumen that circumvents the occlusion [6]. Recanalization rates vary by procedure and embolic agent, ranging from 10% in portal vein embolisation to 15% for pulmonary arteriovenous malformations to 30% for splenic artery embolisation [9,10,11].

US 2007/227544 A1 discloses methods and devices for deployment into a lumen.

WO 2013/025493 A1 discloses systems for the reduction of leakage around medical devices at a treatment site.

WO2014140325 A1 discloses an embolisation device for promoting clot formation in a lumen comprising a stem and a plurality of flexible bristles extending outwardly from the stem, the bristles having a contracted delivery configuration and a deployed configuration in which the bristles extend generally radially outwardly from the stem to anchor the device in a lumen, the device comprising a plurality of segments, each of which comprises a plurality of bristles and wherein the device comprises flexible sections between at least some of the bristle segments. In one configuration a membrane 815 may be placed within the bristle segment such that there are fibres both immediately distal and proximal to the segment.

### Summary of the Invention

In a first aspect, there is provided an embolization device according to Claim 1.

In a second aspect, there is also provided a method of manufacturing according to Claim 13.

### Brief Description of the Drawings

The invention will be more clearly understood from the following description of some embodiments, given by way of example only, with reference to the accompanying drawings, in which:
Fig. 1 is a side view of two connected segments of an embolisation device, in an unconstrained state;
Fig. 2 is an oblique view of proximal segment of the device of Fig. 1;
Fig. 3 illustrates the collapsed configuration of two segments in a catheter, one pointing distally and the other proximally;
Fig. 4 shows the deployed configuration of a device with a proximally pointing proximal segment, a membrane, and a distally pointing distal segment;
Fig. 5 is a schematic of the flow direction (closed arrows) entering a membrane in the deployed configuration and its effect which facilitates the seal against the vessel wall;
Fig. 6 shows the configuration of two distally pointing segments (proximal and distal segments) in the collapsed state;
Fig. 7 shows the configuration of two distally pointing segments (proximal and distal segments) in the deployed state;
Fig. 8 illustrates an unstable device, with poor co-linearity with the vessel centre line which may allow flow to pass through;
Fig. 9 shows the dimensions of the device in the undeployed state (a) and the vessel diameter definition (b);
Fig. 10 illustrates a device with two bristle segments pointing in opposing directions on the same stem;
Fig. 11 illustrates a device with two bristle segments in opposing directions, sharing the same stem and without a gap in between;
Fig. 12 (a) to (c) illustrates a marking system and the location of markers during different stages of delivery and deployment;
Fig. 13 (a) and (b) shows a connection comprising a thread mechanism utilising a twisted wire stem as a natural male thread - in this schematic a formed hypotube is used as the female thread;
Fig. 14 shows a connection comprising a thread mechanism in which a hypotube is attached to a delivery wire and detachable from the twisted wire mechanism by a thread mechanism on the hypotube;
Figs. 15 (a) illustrates an embolisation device in a fully expanded unrestrained configuration and (b) in a deployed configuration;
Fig. 15 (c) is an exploded view of an embolization device;
Figs. 16 (a) and (b) are views similar to Fig. 15 of another embolisation device;
Figs. 17 (a) and (b) are views similar to Fig. 15 of a further embolisation device;
Figs. 18 (a) and (b) are views similar to Fig. 15 of a still further embolisation device;
Fig. 19 is a view of a device of the invention in a packaged configuration ready for use;
Fig. 20 is an enlarged view of a loading tube of Fig. 19;
Fig. 20 (a) is an enlarged view of a distal end of another loading tube;
Fig. 21 is an isometric view of an embolisation device ;
Fig. 22 is an elevational view of an embolisation device of;
Fig. 23 is an isometric view of the device of Fig. 22;
Figs. 24 (a) to (d) illustrate the delivery and deployment of the device of Figs. 22 and 23;
Fig. 25 is an isometric view of a further embolisation device ;
Figs. 26 (a) and (b) illustrate configurations of another embolisation device;
Fig. 27 is an elevational view of portion of a further embolisation device;
Figs. 28 (a) to (c) illustrate the deployment of an embolisation device;
Fig. 29 is an enlarged view of portion of an embolisation device in a deployed configuration;
Fig. 30 a to r illustrates a range of geometries for embolisation devices;
Figs. 31 illustrate an embolisation device with a low profile;
Figs. 32 illustrate another embolisation device;
Figs. 33 illustrate another embolisation device;
Fig. 34 illustrates a further embolisation device including a flow blocking member;
Fig. 35 illustrates further embolisation devices;
Fig. 36 is a diagram of a two segment embolization device bridging an aneurysm;
Fig. 37 is a diagram of a multi segment embolization device bridging a large aneurysm; and
Fig. 38 is a diagram of an embolization device;

### Detailed Description

According to one aspect there is provided an embolization device, comprising:
a proximal segment;
a distal segment, each of the proximal and distal segments including
   a stem, and
   a plurality of anchoring bristles extending outwardly from the stem, wherein the distal segments includes more bristles than the proximal segment; and
   a flow restricting membrane.

In another aspect there is provided an embolization device, comprising:
a proximal segment;
a distal segment, each of the proximal and distal segments including
   a stem, and
   a plurality of anchoring bristles extending outwardly from the stem; and
   a flow restricting membrane located on the proximal segment.

In a further aspect there is provided an embolization device, comprising:
a proximal segment;
a distal segment, each of the proximal and distal segments including
   a stem, and
   a plurality of anchoring bristles extending outwardly from the stem; and
   a flow restricting membrane located longitudinally within the bristles of one of said segments.

In another aspect there is provided an embolization device, comprising:
a proximal segment;
a distal segment, each of the proximal and distal segments including
   a stem, and
   a plurality of anchoring bristles extending outwardly and circumferentially from the stem; and
   a flow restricting membrane extending from the stem and having an outer dimension less than an outer dimension of the plurality of anchoring bristles of the proximal segment.

In a further aspect there is provided an embolization system, comprising:
a delivery catheter; and
an embolization device having a loaded configuration when the device is loaded in the delivery catheter, and a delivered configuration when the device is urged out from the catheter, the embolization device further including
   a proximal segment;
   a distal segment, each of the proximal and distal segments including
   a stem, and
   a plurality of anchoring bristles extending outwardly from the stem, the bristles of the proximal segment being deflected in a first direction in the loaded configuration, and the bristles of the distal segment being deflected in a second direction in the loaded configuration, the first direction being opposite the second direction; and
   a flow restricting membrane deflected in the first direction in the loaded configuration.

According to the present disclosure there is also provided an embolisation device for promoting clot formation in a lumen comprising at least two segments, each segment comprising a stem and a plurality of flexible bristles extending outwardly from the stem, the bristles having a contracted delivery configuration and a deployed configuration in which the bristles extend generally radially outwardly from the stem to anchor the device in a lumen wherein, in the deployed configuration bristles of one segment extend partially in a first longitudinal direction and the bristles of another segment extend partially in a second longitudinal direction which is opposite to the first longitudinal direction.

In one embodiment in the contracted delivery configuration the bristles of one segment extend partially in a first longitudinal direction and the bristles of another segment extend partially in a second longitudinal direction which is opposite to the first longitudinal direction.

In one case the device includes a flow restrictor having a contracted delivery configuration and an expanded deployed configuration. The flow restrictor may be located adjacent to a proximal end of the device. The flow restrictor may be located within or adjacent to the most proximal segment. Alternatively or additionally the flow restrictor is located adjacent to the distal end of the device. The flow restrictor may be located within or adjacent to the most distal segment.

According to the invention, the flow restrictor comprises a membrane.

According to the invention, a flow restricting membrane is located longitudinally within the bristles of the proximal segment and/or the distal segment. The flow restricting membrane may extend from the stem. The flow restricting membrane may have an outer dimension which is less than an outer dimension of the plurality of anchoring bristles. The flow restricting membrane is connected to the stem. According to the invention, the flow restricting membrane has a central hole.

The central hole in the membrane is preferably smaller than the stem on which it is mounted. The central hole in the membrane may have a diameter which is smaller than the diameter of the stem.

The central hole may adapt its shape and dimension at least in part to the shape and dimensions of a cross section of the stem. The central hole may be stretched during mounting in order to fit the stem.

According to the invention, there is an interference fit between the central hole and the stem.

In one case the bristles in an unconstrained configuration extend to a radial extent which is greater than the radial extent of the membrane in the unconstrained configuration. In the constrained configuration, the membrane may have a longitudinal extent. In the deployed configuration, the membrane may have a conical or cup-like shape.

In one embodiment the flow restrictor is of a flexible material. The flow restrictor may be of a polymeric material. The flow restrictor may be of an elastomeric material. The flow restrictor may comprise a film.

In one embodiment the flow restrictor comprises a shape memory material such as Nitinol.

In one case the device comprises connectors between the segments. A proximal connection between the most proximal segment and the segment adjacent to the proximal segment may be relatively stiff. The proximal connection may incorporate or comprise a marker band.

In one embodiment the embolization device comprises only a single proximal segment and a single distal segment. The proximal segment and the distal segment in one case are mounted on a single common stem.

In one embodiment the stem of the proximal segment and the stem of the distal segment form parts of the same continuous stem.

In one embodiment the device comprises a distal marker which is located on the distal side adjacent to the most distal segment.

In one case the device comprises a proximal marker which is located on the proximal side adjacent to the most proximal marker.

In one embodiment the device comprises at least one further segment between a distal segment and a proximal segment. There may be a plurality of further segment between a distal segment and a proximal segment. The connections between at least some of the further segments may comprise hinges to facilitate relative movement between the further segments.

In one embodiment a proximal end of the device is adapted for releasable connection with a delivery means such as a delivery wire or tube. There may be a connector for connection to the delivery wire. The connector may be hingedly moveable relative to the most proximal segment.

In another aspect the present disclosure provides an embolisation device for promoting clot formation in a lumen comprising at least two segments, each segment comprising a stem and a plurality of flexible bristles extending outwardly from the stem, the bristles having a contracted delivery configuration and a deployed configuration in which the bristles extend generally radially outwardly from the stem to anchor the device in a lumen, wherein the device includes a flow restrictor having a contracted delivery configuration and an expanded unrestrained configuration.

In one case the flow restrictor is located adjacent to a proximal end of the device. The flow restrictor may be located within or adjacent to the most proximal segment. Alternatively or additionally the flow restrictor is located adjacent to the distal end of the device. The flow restrictor may be located within or adjacent to the most distal segment.

In one embodiment the flow restrictor comprises a membrane. The bristles in an unconstrained configuration may extend to a radial extent which is greater than the radial extent of the membrane in the unconstrained configuration. In the constrained configuration, the membrane may have a longitudinal extent. In the deployed configuration, the membrane may have a conical or cup-like shape.

In one case the flow restrictor may be of a flexible material. The flow restrictor may be of a polymeric material. The flow restrictor may be of an elastomeric material.
In one embodiment the flow restrictor comprises a film. The flow restrictor may comprise a shape memory material such as Nitinol.

In a further aspect the present disclosure provides an embolisation device for promoting clot formation in a lumen comprising at least two segments, each segment comprising a stem and a plurality of flexible bristles extending outwardly from the stem, the bristles having a contracted delivery configuration and a deployed configuration in which the bristles extend generally radially outwardly from the stem to anchor the device in a lumen, comprising a proximal bristle segment and at least one distal bristle segment, a proximal marker proximal the most proximal segment, a distal marker distal of the most distal segment and an intermediate marker between the most proximal segment and the segment which is adjacent to the most proximal segment.

The present disclosure also provides an embolisation device for promoting clot formation in a lumen comprising at least two segments, each segment comprising a stem and a plurality of flexible bristles which extend radially outwardly of the stem, the bristles having a contracted delivery configuration and a deployed configuration in which the bristles extend generally radially outwardly of the stem, the bristles comprising distal bristles in a distal bristle segment and proximal bristles in a proximal segment and wherein there are differences between at least some of the distal bristles and at least some of the proximal bristles.

In one case the device comprises at least one intermediate segment between the proximal segment and the distal segment, the intermediate segment comprising intermediate bristles and wherein there are differences between the intermediate bristles and either or both of the proximal bristles and the distal bristles.

In one embodiment the differences comprise a difference in radial extent.

At least some of the bristles in the proximal segment may be tapered proximally or distally. Alternatively or additionally, at least some of the bristles in the distal segment are tapered proximally or distally. Alternatively or additionally, the device comprises at least one intermediate segment and at least some of the bristles in the intermediate section are tapered proximally or directly.

In some embodiments at least some adjacent bristle segments are longitudinally spaced-apart.

In one case the differences comprise differences in properties such as flexibility.

The number of distal bristles may be different from the number of proximal bristles.

In some embodiments at least some of the bristle segments are of non-circular profile in the deployed configuration.

The present disclosure also provides an embolization device of the invention and a delivery catheter. In one case the delivery catheter is a microcatheter.

Also provided is an embolisation system comprising:-
an embolisation device having a plurality of bristle segments having a contracted delivery configuration and an expanded deployed configuration;
a connector at a proximal end of the embolisation device;
and a delivery element which is releasable connected to the connector for delivery of the embolisation device into the expanded deployed configuration.

The connector may be configured to facilitate movement between the delivery element and the embolisation device. The connector may be hingedly mounted to the embolisation device.

In one case the system further comprises a delivery catheter in which the embolisation device is retained in the retracted configuration.

Also provided is an embolization device comprising:-
a proximal segment;
a distal segment; and
a flow restricting member, each of the proximal and distal segments including a stem and
a plurality of anchoring bristles extending outwardly from the stem.

The flow restrictor may comprise a membrane. The flow restricting membrane may be located on the proximal segment. The flow restricting membrane may be located on the distal segment.

In one case a flow restricting membrane is located longitudinally within the bristles of the proximal segment and/or the distal segment. The flow restricting membrane may extend from the stem. The flow restricting membrane may have an outer dimension which is less than an outer dimension of the plurality of anchoring bristles. The flow restricting membrane may be connected to the stem. In some cases the flow restricting membrane may have a central hole. The central hole in the membrane is preferably smaller than the stem on which it is mounted. The central hole in the membrane may have a diameter which is smaller than the diameter of the stem.

In one case the central hole adapts its shape and dimension at least in part to the shape and dimensions of a cross section of the stem. The central hole may be stretched during mounting in order to fit the stem.

In one case there is an interference fit between the central hole and the stem.

In one case the flow restricting membrane is not attached to the plurality of bristles.

The flow restricting membrane may be substantially impermeable.

The flow restricting membrane may have a contracted delivery configuration and an expanded deployed configuration. In the constrained configuration, the flow restricting membrane may have a longitudinal extent. In the deployed configuration the flow restricting membrane may have a conical or cup-like shape.

The flow restrictor may be of a flexible material. The flow restrictor may be of a polymeric material. The flow restrictor may be of an elastomeric material. The flow restrictor may comprise a film.

In one case the flow restricting membrane is more flexible than the bristles adjacent to it.

In one embodiment the distal segment includes more bristles than the proximal segment.

The diameter of the bristles in the distal segment may be greater than the diameter of the bristles in the proximal segment.

In one case the stem of the proximal segment is mounted to the stem of the distal segment. The stem of the proximal segment may be substantially rigidly mounted to the stem of the distal segment.

The embolization device in some cases further comprises at least one radiopaque marker. There may be a radiopaque marker adjacent to a distal segment and/or a radiopaque marker adjacent to the proximal segment and/or a radiopaque marker intermediate the proximal and distal segments.

In one embodiment the device comprises a proximal connector for releasable connection to a delivery element. The proximal connector may comprise a stem portion. The connector stem may be coupled to the proximal segment stem. The connector stem may be hingedly mounted to the proximal segment stem. The connector stem may have a mounting feature for engagement with a mounting feature of a delivery element. The connector mounting feature may comprise a screw thread.

In one embodiment the embolization device comprises only a single proximal segment and a single distal segment. The proximal segment and the distal segment in one case are mounted on a single common stem.

In one embodiment the stem of the proximal segment and the stem of the distal segment form parts of the same continuous stem.

The embolization device may comprise at least one further segment between the distal segment and the proximal segment. There may be a plurality of further segment between a distal segment and a proximal segment. The connections between at least some of the further segments may comprise a hinge to facilitate relative movement between the further segments. The connection between some of the segments intermediate the proximal segment and the distal segment may be relatively rigid.

In one case the proximal segment comprises from 40 to 150 bristles, in one case 60 to 150, optionally 70 to 110, optionally about 90, in another case 50 to 110, optionally 70 to 90, optionally about 80, in another case 40 to 100, optionally 40 to 75, optionally 40 to 60.

In one case the distal segment comprises from 40 to 180 bristles, in one case 70 to 180, optionally 100 to 150, optionally about 125, in another case 50 to 130, optionally 80 to 100, optionally about 90, in another case 40 to 80, optionally 40 to 60.

Also provided is method for manufacturing an embolization device comprising the steps of:-
providing a bristle segment having a plurality of bristles extending outwardly of the stem;
providing a bristle manipulating tool;
manipulating at least some of the bristles so that the bristles are aligned with the stem;
mounting a flow restrictor membrane between the bristles; and
releasing the bristles from the manipulating tool.

According to the invention, the method further comprises the step of mounting the membrane on the stem of the bristle segment.

In one case the membrane comprises a central hole which is smaller than the diameter of the bristle stem and the method comprises engaging the stem in the hole of the membrane.

In one case the central hole adapts its shape and dimension at least in part to the shape and dimensions of a cross section of the stem. The central hole may be stretched during mounting in order to fit the stem.

According to the invention, there is an interference fit between the central hole and the stem.

Referring to the drawings there is illustrated an embolisation device 1 which comprises a plurality of flexible bristles having deployed and contracted configurations. The device comprises a series of segments wherein at least one segment 3 points distally and one segment 4 points proximally. In some cases there is only a proximal segment 4 and distal segment 3.

The bristles of the proximal segment 4 point proximally and the bristles of the distal segment 3 point distally.

A proximally pointing segment is defined as a segment in which the bristles point proximally and the membrane (if present) cone is open at the proximal end. A distally pointing segment is defined as a segment in which the bristles point distally and the membrane (if present) cone is open at the distal end.

At least one segment in this case the proximal segment 3, incorporates a flow restrictor which in this case is a thin film flexible membrane 5.

In some cases a series of radiopaque markers divides the proximally pointing segment 4 and the distally pointing segment 3. There may be a proximal marker 6, a distal marker 7 and an intermediate marker 8.

In one case the embolization device comprises only a single proximal segment 4 and a single distal segment 3. The proximal segment 4 and the distal segment 3 in one case are mounted on a single common stem. The stem of the proximal segment 4 and the stem of the distal segment 3 may form parts of the same continuous stem.

In the case where the device comprises more than two segments, the connection between the two most proximal segments is more stiff than the distal connections. The distal connections generally comprise a hinge.

In one embodiment, a flexible membrane 5 is present in at least one of the segments. The membrane 5 may comprise a disc of thin film material. The flexibility of the membrane 5 means its orientation is controlled by the orientation of the adjacent bristles - i.e. if the adjacent bristles are forced to point distally the membrane 5 will adjust its configuration accordingly. Thus, if the membrane 5 is deployed from a collapsed condition, such as from within a catheter, the bristles will cause it to open up to an expanded configuration. The membrane 5 may also be placed proximal or distal to the segment.

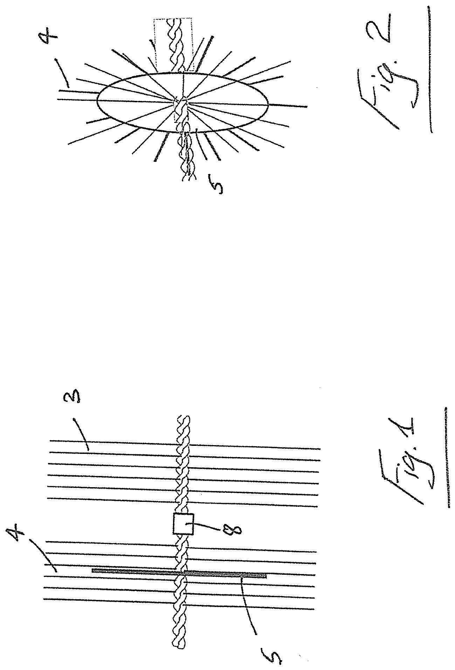

In one case, the implant device comprises at least two segments. In one configuration the membrane 5 is in the most proximal segment. This is shown, in an unconstrained state schematically in Figs. 1, 2, 21, 22 and 23. In the configuration shown the membrane 5 is located within the proximal segment 4 with bristles both proximal and distal to the membrane 5. In some cases there may be a distal membrane 5'.

In one case a flow restricting membrane is located longitudinally within the bristles of the proximal segment and/or the distal segment. The flow restricting membrane may extend from the stem. The flow restricting membrane may have an outer dimension which is less than an outer dimension of the plurality of anchoring bristles. The flow restricting membrane may be connected to the stem. In some cases the flow restricting membrane may have a central hole that is an interference fit on the stem. The central hole in the membrane is preferably smaller than the stem on which it is mounted. The central hole in the membrane may have a diameter which is smaller than the diameter of the stem.

The implant has a collapsed configuration to facilitate delivery through a catheter. By placing the membrane 5 within the segment 4, i.e. with bristles proximal and distal to it, it is protected from damage while the implant is being collapsed, or pushed through a catheter. Furthermore, any friction between the catheter and the membrane 5 is reduced.

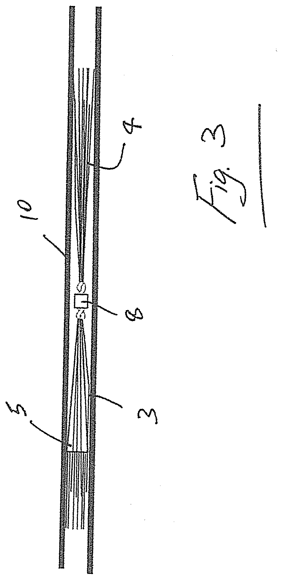

In one configuration, the implant is collapsed such that, the bristles of the most proximal segment 4 point proximally, while the bristles of the distal segment 3 or segments point distally. Since the membrane orientation is controlled by the orientation of the bristles, if the membrane 5 is within the proximal segment, it will also point proximally. This is shown schematically in Fig. 3.

Fig. 3 shows a collapsed configuration of two segments 3,4 in a catheter 10, one pointing distally and the other proximally. It will be noted that the outer periphery of the membrane 5, shown in the proximal segment 4, is pointing proximally.

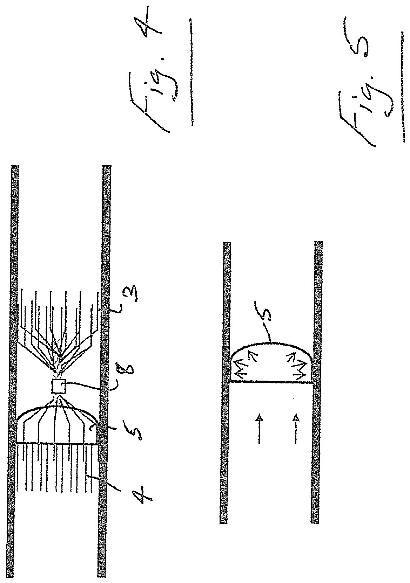

When deployed from this configuration, into a vessel a similar but partially expanded configuration to the collapsed configuration is achieved. This means that the bristles of the proximal segment 4 point proximally, and the bristles of the distal segment 3 point distally. This is shown schematically in Fig. 4. In this configuration the implant will be anchored from moving in either direction. This is because the ends of the bristle act in a brake-like fashion increasing friction between the implant and the wall. On the contrary, if all bristles point distally, the force required to push the implant distally will be greater than that required to push the implant proximally. Thus a device migration may be more likely to occur in the proximal direction.

In one embodiment the membrane 5, when measured in the unconstrained configuration, has a diameter which is less than that of the bristle segment, but greater than that of the vessel for which the device is intended. Thus the membrane is sufficiently large in diameter to contact the circumference of the vessel. A larger membrane would increase the profile of the implant when in the collapsed condition necessitating a larger catheter for delivery.

As illustrated in Fig. 4 when the device is deployed into a vessel, with a smaller diameter than the implant, the membrane 5 assumes a conical or cup-like shape - the open end of the cone proximal to the closed end. In one configuration the deployed implant comprises a membrane 5 with a conical shape, the open end of the cone proximal to the distal end. In arteries blood from the heart towards the distal arterial tree, that is from proximal to distal. The configuration ensures that the blood flows into the cone's volume, i.e. the opening of the cone opposes flow. Thus the blood will act to expand the cone further enhancing the seal between the membrane and the vessel wall (Fig. 5). In this way occlusion will be facilitated. Thus the greater the force (pressure) of the flow into the cone, the greater the improvement of the seal against the vessel wall.

Fig. 5 shows a schematic of the flow direction (closed arrows) entering a membrane 5 in the deployed configuration and its effect on the seal against the vessel wall.

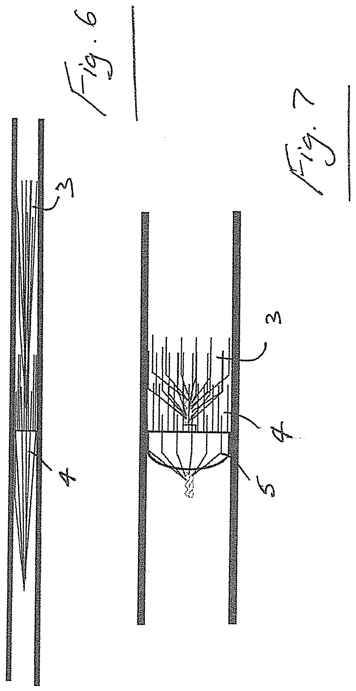

In another embodiment the implant may be collapsed such that all segments point distally. Figs. 6 and 7 show the configuration of two distally pointing segments 3,4 (proximal and distal segments) in the collapsed state. When deployed, both the proximal and distal segments will point distally. Similarly, all segments may be collapsed such that all point proximally. This may be advantageous when attempting to occlude a lumen in which flow is from distal to proximal, such as a healthy vein. Figs. 6 and 7 show the configuration of two distally pointing segments (proximal and distal segments) in the collapsed and deployed state.

A different degree of under sizing of the membrane with respect the segment diameter may be preferable for devices intended for arteries and veins. For example veins are known to distend more than arteries during manoeuvres such as Valsalva. Typically arteries distend by 5 to 15% while veins can distend 20-60%.

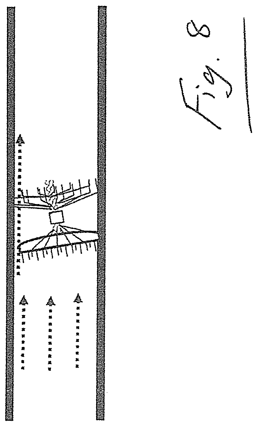

To ensure an adequate seal between the membrane 5 and the vessel wall it is preferable that the segment centreline is co-linear with that of the vessel. Use of at least two segments in the device pointing in opposite directions helps to remedy this problem, i.e. the bristles of the proximal segment pointing proximally, and the bristles of the distal segment pointing distally. This facilitates a uniform seal of the cone against the vessel wall about its circumference. As can be seen in Fig. 8, if the segment is not co-linear with the vessel, the membrane may be unstable. This instability may enable flow to open or alter the membrane geometry from a cone-like shape (for example by flipping the direction of the cone). Fig. 8 illustrates an unstable device, with poor co-linearity with the vessel centre line which may allow flow to pass through.

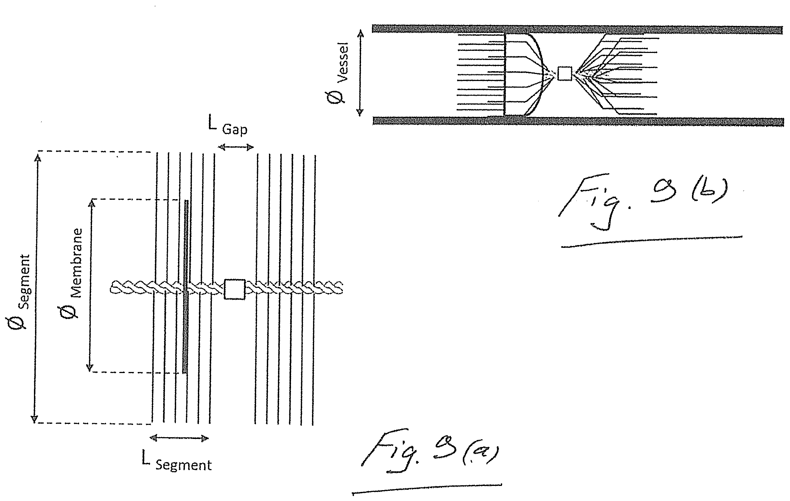

The device may include features to improve co-linearity of the device with the vessel centreline. In one embodiment, the diameter of the segment is significantly larger than that of the target vessel. This improves the stability of the device within the vessel facilitating co-linearity of the segment and the vessel centreline. Thus the implant is significantly oversized compared to the target vessel. Preferable dimensions are outlined in Table 1 and Table 2 for devices deliverable through 0.0385 to 0.041 inch, and 0.056-0.056 inch inner diameter catheters respectively. The dimensions are shown schematically in Fig. 9. Fig. 9 shows the dimensions of the device in the undeployed state (a) and the vessel diameter definition (b).

The oversizing (calculated as the percentage difference in diameter between segment diameter and the vessel diameter) is preferably at least 20%, more preferably 50% of the vessel diameter and more preferably at least 100% of the vessel diameter in which the device is implanted. For example for a target vessel which is 6 mm in diameter, the device diameter may be at least 7.6mm, preferably at least 9mm, more preferably at least 12mm.

To ensure co-linearity in veins, the degree of oversizing may be increased compared to that used in arteries. This is because veins are known to distend significantly (for example during Valsalva). In one configuration the minimum over-sizing is 100%.

In one configuration the connection between two segments has some flexibility to enable tracking through tortuous anatomy or to accommodate vessel movement during waking etc. It is preferable that the flexibility of this connection is limited so as to ensure good co-linearity of the segment with the membrane and the vessel ensuring good vessel occlusion. This prevents the device from deploying in a buckled configuration as it exits the catheter tip.

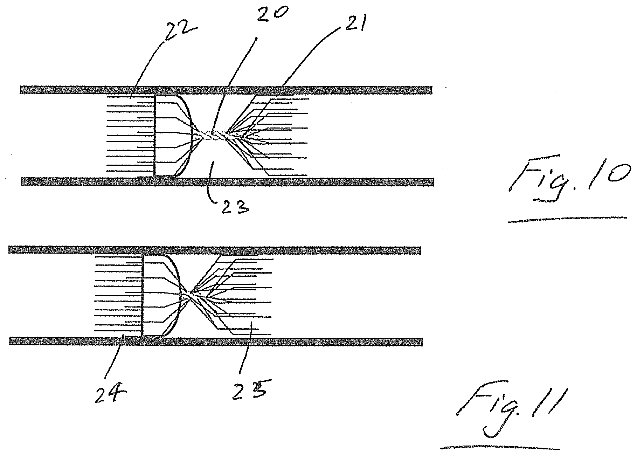

In one embodiment, the bristle segments are on the same stem 20 and there is a gap 23 between the segments 21, 22 as illustrated in Fig. 10. In another embodiment, two segments 24, 25 on two different stems may be connected. In one configuration this connection comprises a crimped or welded hypotube. Fig. 10 shows a device with two bristles segments 21, 22 pointing in opposing directions on the same stem 20.

In yet another embodiment, the same segment may be configured in the collapsed and deployed configuration so as to have some bristles (and the membrane) pointing proximally and some bristles pointing distally (Fig. 11). Fig. 11 shows a device with two bristle segments 24, 25 in opposing directions, sharing the same stem and without a gap in between.

In some instances the physician may wish to deploy at least a portion of the device and reposition it if he or she is not satisfied.

In one case the device incorporates at least one proximally pointing proximal segment, and at least one distally pointing distal segment. If the physician deploys the device completely and then wishes to retrieve and redeploy the device, the action of retrieving the implant by advancing the guide catheter over it will cause the direction of the proximal segment or segments to flip upon passing through the catheter tip. Thus if the implant is re-deployed all segments will point distally. This will cause the membrane to be open distally. Thus flow may be able to flow past the outside of the membrane. It may be preferable to avoid this situation.

To mitigate this, a radiopaque marking system may be utilised to alert the physician of whether the proximally pointing segment or segments have been deployed from the catheter. Thus the physician can deploy the distally pointing segments, and assess their position without deploying the proximally pointing segment or segments. If the physician is unhappy with the position of the distally pointing segments, they may resheath and redeploy them without altering the direction of the proximally pointing segments.

In one configuration a radiopaque marker, distal marker 7, is present at the most distal point of the most distal segment. A second marker, medial marker 8, is present between the distally pointing segment(s) and proximally pointing segment(s). A third marker, proximal marker 6, is present at the most proximal point of the proximally facing segment. In this configuration the section between the distal and medial marker 7,8 defines the distally facing segments which may be deployed, retrieved/repositioned without any effect on their pointing direction, and the section between the medial and third proximal 6,8 defines the proximally facing segment which should not be deployed until the physician is happy with position of the device.

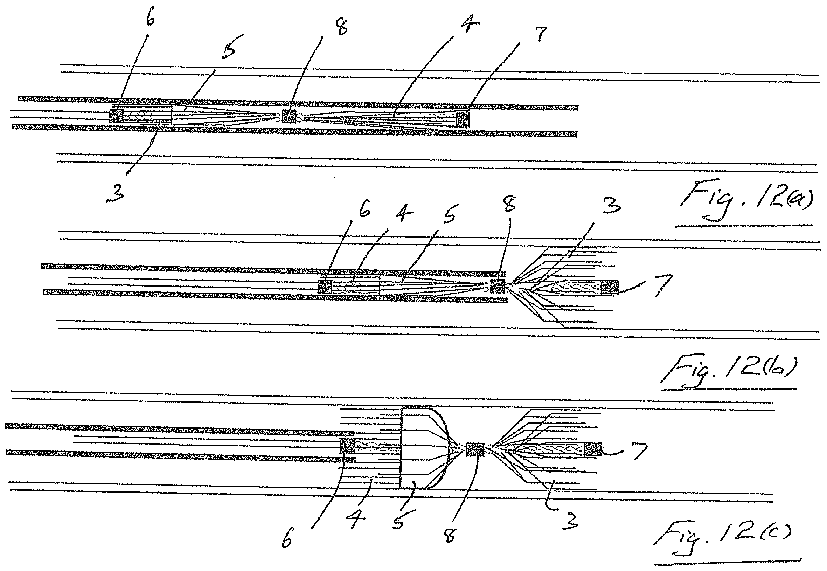

The deployment of the device using this marking system is shown schematically in Figs. 12(a) to (c) which show the marking system and their locations during different stages of delivery and deployment.

In one case, a section of tube of a radiopaque material known as a marker band may be crimped onto the connection between the segments. In the case in which a hypotube is used to connect the segments, the marker may be placed on one or both of the stems of the segments before the hypotube is crimped in place. In another embodiment the marker band may be placed onto the hypotube connection. In yet another embodiment the radiopaque marker band may be used to connect two adjacent segments. Attachment may be facilitated by crimping or welding, soldering, use of an adhesive or other means. In another configuration a marker band may be placed on the stem distal or proximal to the connection between the two segments.

In one embodiment the membrane is made from a thin film of PTFE. In one embodiment the membrane is made from a thin film elastomer such as polyurethane. In one case the membrane is of a thermoplastic polyurethane, such as a poly-ether urethane, for example an aromatic polyether urethane. In one embodiment the membrane incorporates a small hole at its centre. To facilitate placement of the membrane on the bristle segment, the adjacent bristles are collapsed by some means. The membrane can then be threaded over the collapsed bristles into the desired position.

Manipulating the membrane over the collapsed bristles into position may require that the hole is stretched to a larger diameter. The use of an elastomer which can accommodate larger deformations without permanently deforming facilitates this step in manufacture facilitates this. The ability of this material to stretch facilitates placement of the membrane within the segment during manufacture.

Because a material such as polyurethane is less lubricious than others ensuring that the membrane is adequately held against the bristles of the segment in the collapsed configuration and cannot be pulled off during loading and delivery through a catheter.

In one embodiment the membrane is made from thin film Nitinol. In this instance the bristles are not required to collapse, expand and support the membrane.

Preferably the membrane has a low stiffness. This ensures that its behaviour is dominated by the bristles by the adjacent bristles, and that it can easily flex to ensure a good seal at the vessel wall. Furthermore a stiff membrane may have channels longitudinally. Another problem with a stiff membrane is that cannot fold and conform to a low profile when in the collapsed configuration. Considering the situation where a polymer membrane such as polyurethane is used, the stiffness of the membrane may be reduced by reducing its thickness to that of thin film. Dimensions for the membrane are outlined in Table 1 and Table 2. The membrane may also be of PTFE, PET, or Nylon. PTFE is particularly suitable as it will enhance lubricity enabling the device to be delivered through the catheter without high force.

It is preferable that the device profile when in the collapsed configuration is as low as possible in order to enable delivery through a small bore catheter. This reduces complications such as hematoma and infection at the site of luminal access for the catheters. It is also preferable that the implant be detachable from the delivery wire at the discretion of the physician.

In one configuration, the implant has a detachment mechanism on its proximal end. This ensures that the physician can readjust its position until he or she is happy, remove the device, or detach the implant at will. For some designs the diameter of the detachment mechanism may exceed that of the stem, or even fill the majority of the space within catheter or sheath used to deliver the implant to the target vessel. Accordingly, when in the collapsed state, if the bristles or membrane overlap the detachment mechanism an increased or excessive profile may occur.

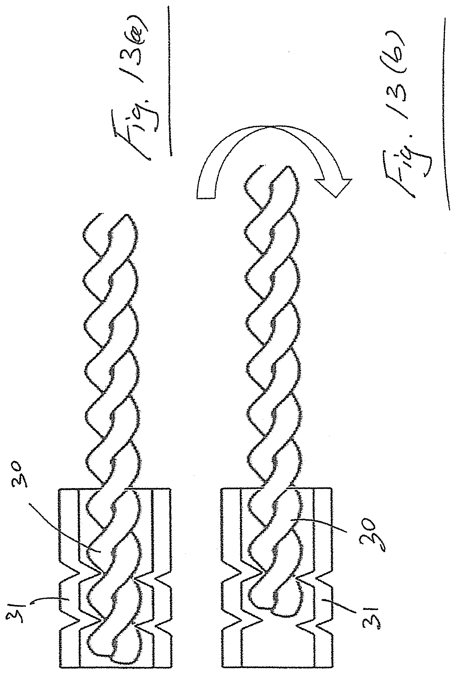

Another solution to this problem is to use a low profile detachment mechanism. In one embodiment, a twisted wire stem may be used wherein the geometry of the twisted wire naturally provides a male screw thread as shown in Fig. 13 A female screw thread may then be threaded onto this male screw thread. In one embodiment the female screw thread mechanism comprises a formed hypotube in which the threads on the inside of the hypotube intended to mate with the threads of the twisted wire stem are formed in place. Preferably the pitch of the twisted wire brush is the pitch of the thread. A relatively low number of threads may be used with success. A minimum of two female threads is used preferably.

Fig. 13 shows a thread mechanism utilising the twisted wire stem 30 as a natural male thread. In this schematic a formed hypotube 31 is used as the female thread.

In another embodiment the female screw thread is machined or tapped onto the inside of a tube. In another configuration the female screw thread comprises a coil with a pitch to match that of the male thread providing a reliable screw detachment mechanism.

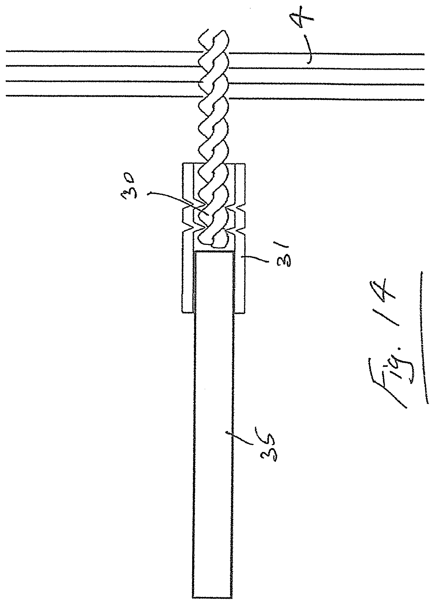

In one embodiment the male screw thread 30 is a section of the twisted wire stem of the most proximal segment. In a further embodiment, the female thread or hypotube 31 is attached at its proximal end to a delivery wire 35. This facilitates delivery and detachment of the bustle segment through a catheter. This is illustrated schematically in Fig. 14 which shows a thread mechanism in which a hypotube 31 is attached to a delivery wire 35 and detachable from the twisted wire stem 30 by a thread mechanism. A thread mechanism which does not utilise the twisted wire stem may also be utilised.

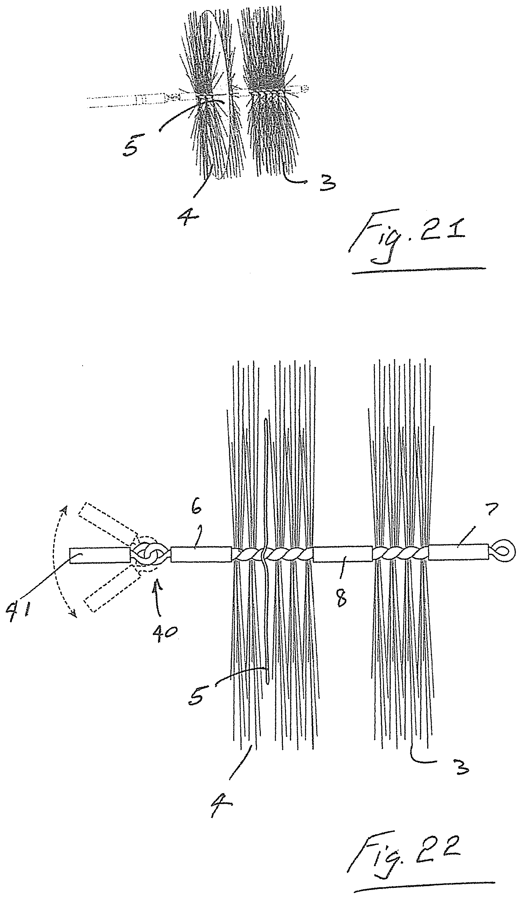

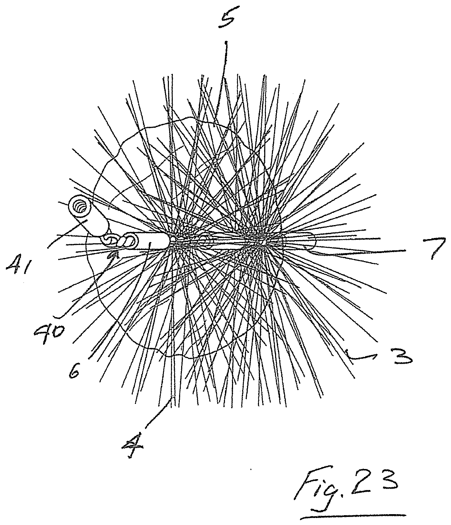

Referring in particular to Figs. 22 and 23 in one case a flexible section 40 is provided between the screw detachment mechanism 41 and the most proximal segment 4 of the implant. In one embodiment this flexible section 40 is a hinge. This flexible section 40 enables delivery and detachment of the implant in tortuous anatomies. This flexible section 40 also serves to ensure that the proximal end of the implant is atraumatic.

In one embodiment the membrane and bristles do not overlap the detachment mechanism. In this case the detachment mechanism is located a minimum distance from the most proximal point of the segment such that the bristles and membrane do not, or at least minimally overlap the detachment mechanism.

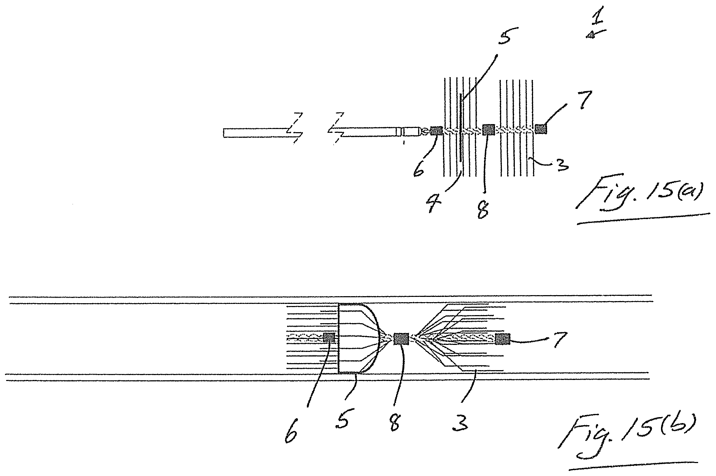

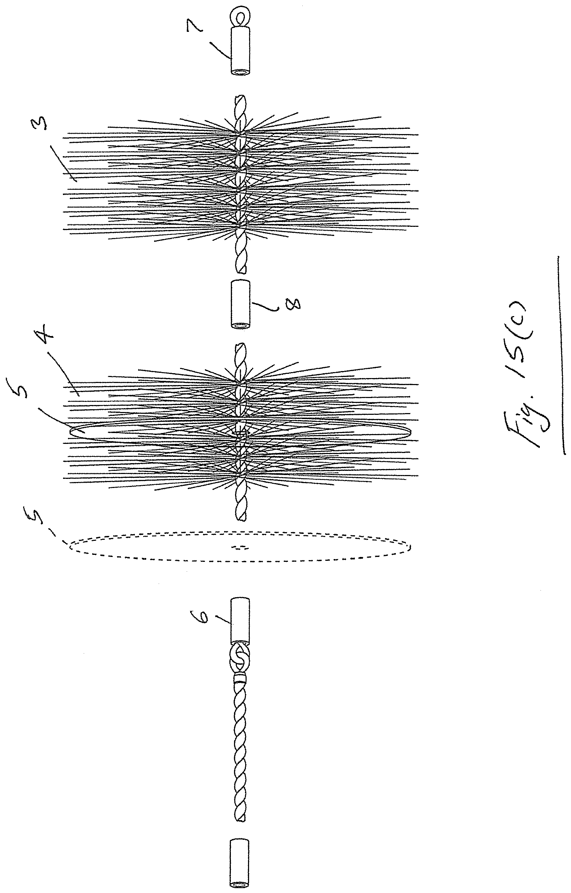

A number of potential device configurations are shown in Figs. 15 to 18. In Figs. 15(a) and (b) there are two segments - one proximal containing the membrane 4 and one distal. Marker bands 6,7,8 are positioned as described above.

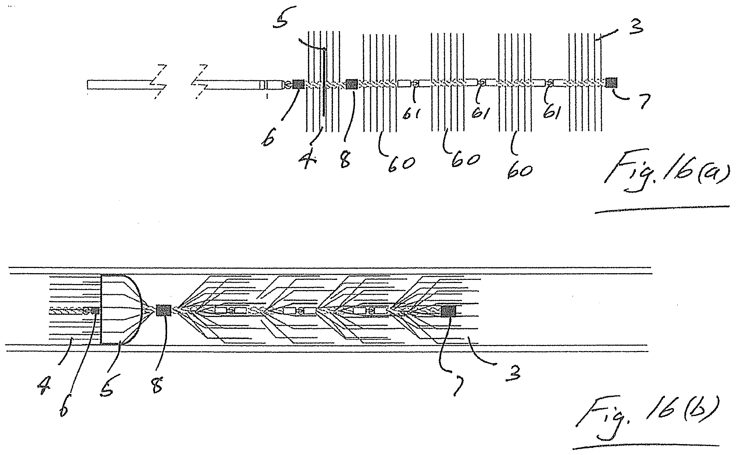

Referring to figs 16(a) and (b) in this case there are additional distal segments 60 and hinge connections 61 are provided between the distal segments to accommodate movement between the segments.

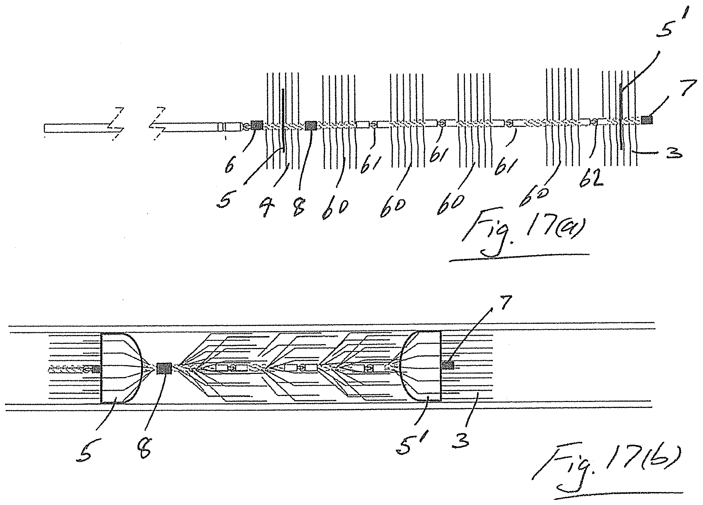

Referring to Figs. 17(a) and (b), in this case the most distal segment also includes a membrane 5' which has an opening which faces distally in the deployed configuration. A relatively stiff connection 62 is provided between the most distal segment and the adjacent segment.

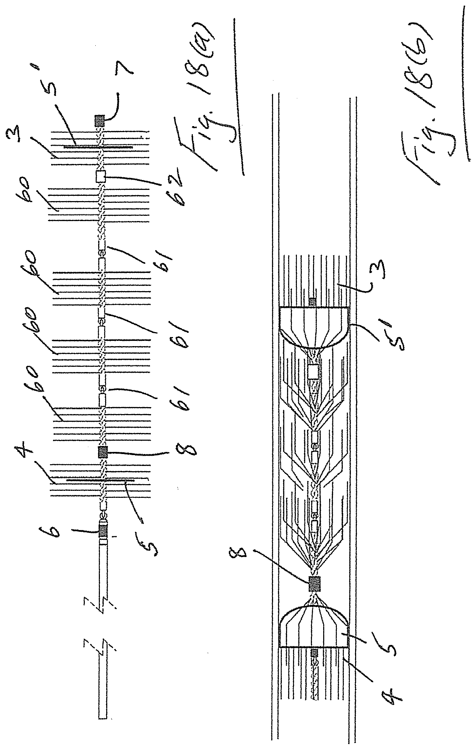

Figs. 18(a) and (b) illustrate a device similar to Fig. 17 and again for increased stability when deployed, there is a relatively stiff connection 62 between the most distal segment and the adjacent segment. The connection in this case may be reinforced by or provided by a section of hypotube.

Figs, 19 and 20 show the complete device configuration. In the packaged configuration, when ready for use, the implant is stored within a loading tube 50. This loading tube 50 comprises a tube with a haemostasis valve 52 and side arm 51 for flushing. The delivery wire 55 is attached to the proximal end of the implant and passes through the haemostasis valve. The implant can be pushed from the loading tube 50 into a catheter for delivery to the target site. In one embodiment the loading tube has a taper at its distal end to enable it to easily fit into the luer of the catheter used for delivery of the device to the target vessel.

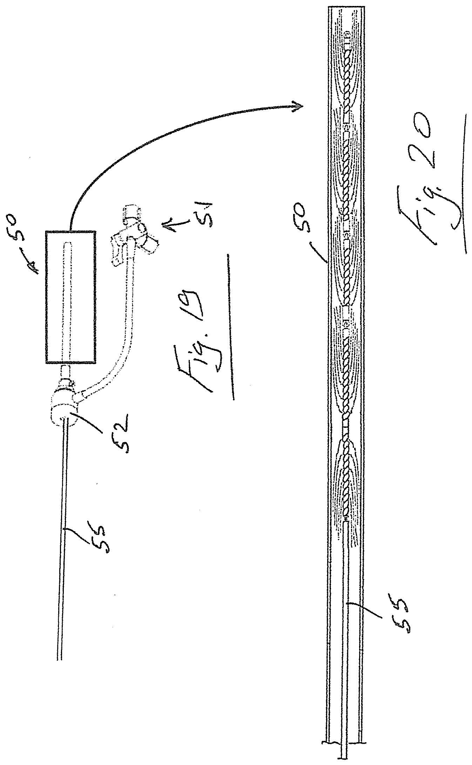

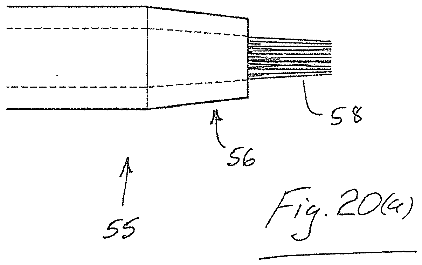

As previously described the implant is pushed from a loader into a catheter to be pushed to the site of treatment. An example of the loader is shown in Fig. 20. In one embodiment the loading tube is made from a lubricious material such as PTFE with an outer diameter of approximately 2.9mm and an inner diameter of approximately 1.65mm. This loading tube is compatible with both 0.056-0.057" 5F delivery catheters and 0.035"-0.038" 4Fr delivery catheters (1.42-1.44 mm and 0.89-0.97 mm).

In another embodiment the loading tube has a taper at its distal end to enable it to be compatible with multiple catheters of differing hub geometries used for delivery of the device to the target vessel. A taper 56 on the loading tube 55 functions by funnelling the bristles 58 of the distal segment of the implant into a conical shape. On exit from the loader, the bristles funnel to a profile less than that of the inner diameter of the catheter hub ensuring that the implant can be pushed freely from the loading tube without snagging. This allows smooth transition across the tube/catheter interface and within the delivery catheter.

In one preferred embodiment the outer diameter of the loading tube is 2.9mm and the inner diameter is 1.65mm. The distal taper comprises an inner diameter from 0.8mm - 1.3mm tapered over a length of 1 - 6 mm.

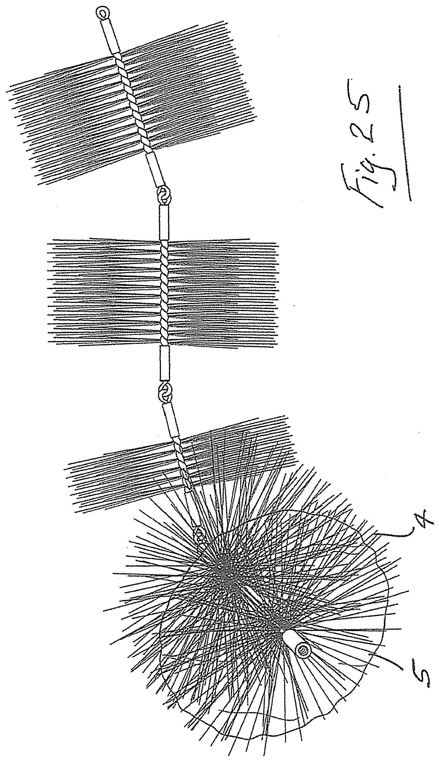

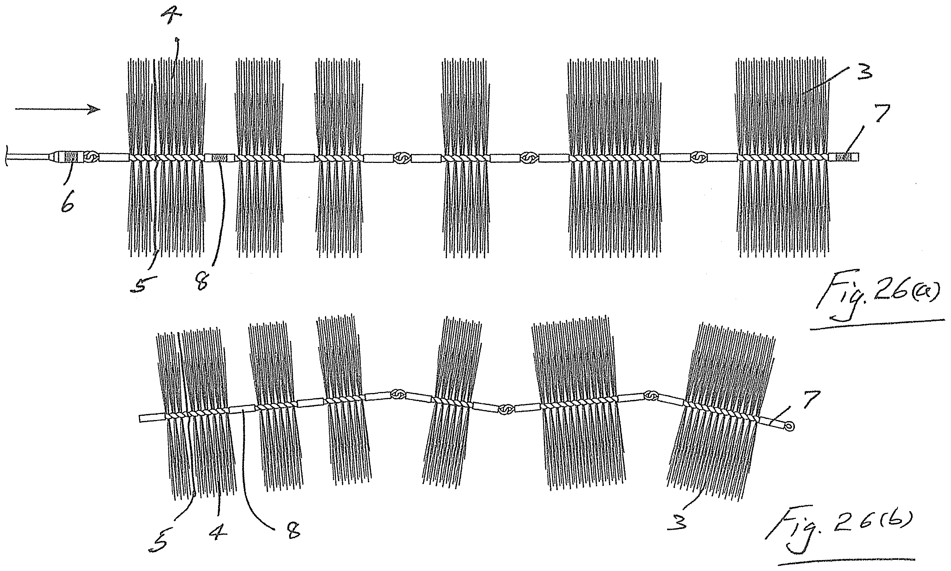

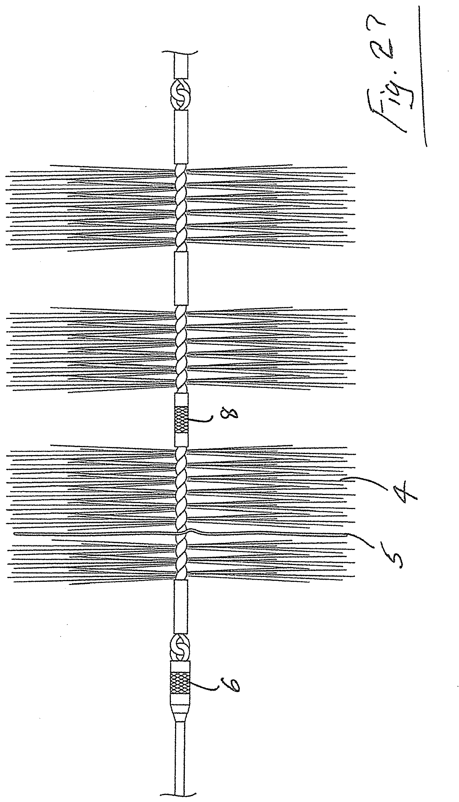

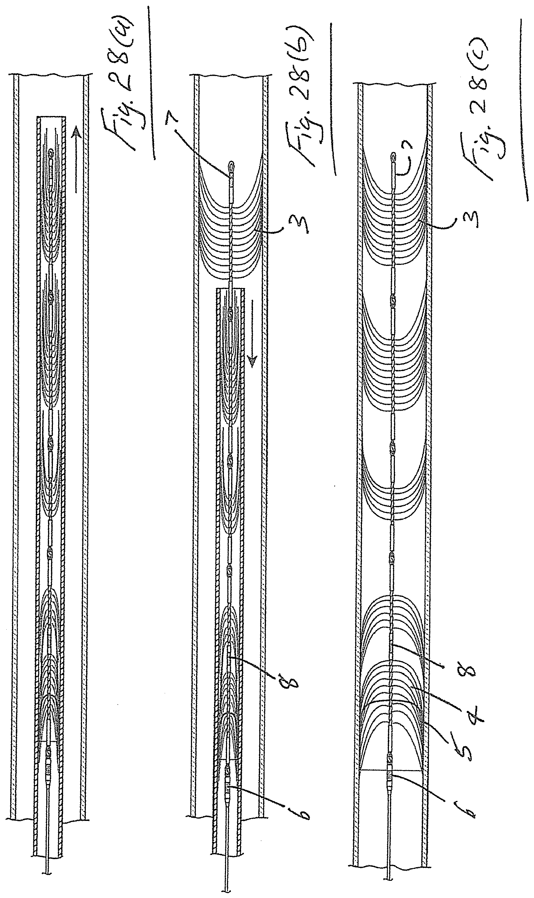

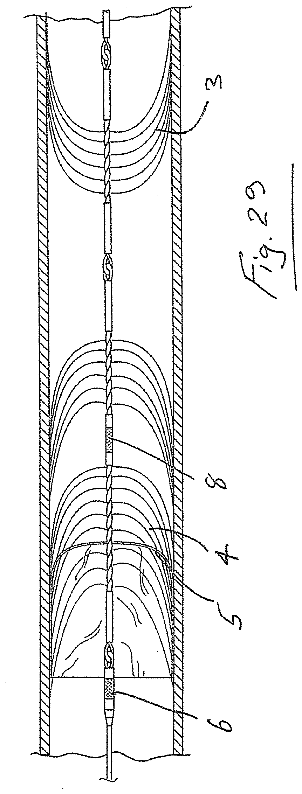

Various configurations of segments, membranes and connections are illustrated in Figs. 25 to 29. In some instances, it may be preferable due to space restrictions within the delivery catheter, to incorporate a different number of bristles within the proximal and distal bristle segment. This enables he number of bristles which encourage thrombus formation and prevent device migration to be maximised, while preventing excessive friction within the catheter during delivery and deployment. This is particularly important in the case in which one bristle segment of the implant incorporates a membrane since the membrane itself will take up space. It is also preferable to minimise the diameter of the stem to further enable addition of more bristles. The stem wire preferably has sufficient diameter to ensure that when twisted the bristles are securely held via plastic deformation of the stem wire. The following tables contain preferable combinations of materials and dimensions for the implant. In the following tables and examples, 1 inch corresponds to 25.4 mm.

**Table 1**

| **Attribute** | **Materials** | | |
|---|---|---|---|
| | **Range** | **Preferably** | **More Preferably** |
| **Bristle Material** | Any shape memory metal or polymer | - | Nitinol, Elgiloy, Nitinol |
| **Stem Wire Material** | Stainless Steel, Cobalth Chromium, Platinum, Tantalum Titanium | Cobalt Chromium or Nickel Alloy | Elgiloy, L605 or MP35N |
| **Membrane Material** | PTFE, PEEK, Polyurethane, Polyether urethane, Polyester urethane, Polycarbonate urethane | - | Polyether urethane 80A |
| **Stem Wire Material Condition** | - | - | Annealed |

**Table 2a**

| **Attribute** | **0.035-0.040in ID Catheter** | | |
|---|---|---|---|
| | **Preferably ≥ 0.038in** | | |
| | | | |
| | **Implant for short vessel treatment** | | |
| | **Range** | **Preferably** | **More Preferably** |
| **Length of Implant (cm)** | 1-20 | 1-6 | 1.5-2.5 |
| **Suitable Artery Diameter (mm)** | 2-13 | 3-10 | 3-7 |
| **Suitable Vein Diameter (mm)** | 2-10 | 3-8 | 3-8 |
| **Bristle Diameter (in)** | 0.001-0.002 | 0.0015-0.0018 | 0.00175 |
| **Number of Segments** | 2-15 | 2-15 | 2 |
| **Number of Bristles in Proximal Segment** | 50-130 | 70-90 | 80 |
| **Length of Proximal Bristle Segment (mm)** | 2.5-4.0 | 2.9-3.5 | 3.5 |
| **Number of Bristles in Distal Segment (no. per mm)** | 60-140 | 90-110 | 100 |
| **Length of Distal Bristle Segment (mm)** | 3.0-5.0 | 3.7-4.5 | 4.4 |
| **Membrane Diameter (mm)** | 6-14 | 6-10 | 8 |
| **Membrane thickness (µm)** | < 25 | < 18 | 8-16 |
| **Membrane Location** | Proximal and distal segment | Proximal and distal segment | Proximal segment |
| **Stem Wire Diameter (in)** | 0.004-0.010 | 0.005-0.008 | 0.006 |
| **Distal and Proximal Segment Diameter (mm)** | 7-20 | 10-18 | 15 |
| **Gap between segments (mm)** | 0.5-10 | 2-5 | 3-4 |
| **Direction of fibres in most proximal segment** | Proximally pointing | Proximally pointing | Proximally pointing |
| **Direction of fibres in most distal segment, or segments** | Distally pointing | Distally pointing | Distally pointing |

**Table 2b**

| **Attribute** | **Implant for Treatment of Short Vessel Segments** | | |
|---|---|---|---|
| | | | |
| | **0.054-0.060in ID Catheter** | | |
| | **Preferably 0.056in** | | |
| | **Range** | **Preferably** | **More Preferably** |
| **Length of Implant (cm)** | 1-30 | 1.5-9 | 2.0-3.5 |
| **Suitable Vein Diameter (mm)** | 2-14 | 3-13 | 5-11 |
| **Suitable Artery Diameter** | 3-10 | 4-9 | 5-8 |
| **Number of Bristle Segments** | 1-30 | 1-25 | 2 |
| **Number of Bristles in Proximal Segment** | 60-150 | 70-110 | 90 |
| **Length of Proximal Bristle Segment (mm)** | 2-6 | 4-5 | 4.10± 0.5mm |
| **Number of Bristles in Distal Segment** | 70-180 | 100-150 | 125 |
| **Length of Distal Bristle Segment (mm)** | 2-10 | 5-7 | 5.75± 0.5mm |
| **Membrane Diameter (mm)** | 11-20 | 13-15 | 14 |
| **Membrane thickness (µm)** | < 25 | < 18 | 810-16 |
| **Membrane Location** | Proximal and or distal segment | Proximal and distal segment | Proximal segment |
| **Stem Wire Diameter (in)** | 0.004-0.010 | 0.005-0.008 | 0.006 |
| **Bristle Diameter (in)** | 0.001-0.0025 | 0.00175-0.002 | 0.002 |
| **Segment Diameter (mm)** | 14-38 | 18-30 | 25 |
| **Gap Between Segments** | 0.5-10 | 2-5 | 3-4 |
| **Direction of fibres in most proximal segment** | Proximally pointing | Proximally pointing | Proximally pointing |
| **Direction of fibres in most distal segment, or segments** | Distally pointing | Distally pointing | Distally pointing |

In some instances it may be preferable to use a much longer device for vessel occlusion. For example, in the case of gonadal veins, devices from 5cm to 15cm, or even 25cm may be required to treat the entire vessel length. For such a vessel, a lower bristle diameter may be appropriate even in a large vessel (e.g. 10mm diameter) since the increased number of bristles, due to the increased length and number of segments, means a sufficient anchor force can be achieved. A reduced bristle diameter in combination with a larger number of bristles enables a lower force for advancement through a catheter, and deployment from a catheter. The following table outlines some preferable combinations.

**Table 3a**

| **Attribute** | **0.054-0.060in ID Catheter** | | |
|---|---|---|---|
| | **Preferably 0.056in** | | |
| | **Range** | **Preferably** | **More Preferably** |
| **Length of Implant (cm)** | 1-30 | 1.5-9 | 2.0-3.5 |
| **Suitable Vein Diameter (mm)** | 2-14 | 3-13 | 3-12 |
| **Number of Bristle Segments** | 2-30 | 4-25 | 2 |
| **Number of Bristles in Proximal Segment** | 60-150 | 70-110 | 90 |
| **Length of Proximal Bristle Segment (mm)** | 2-6 | 4-5 | 4.5 |
| **Number of Bristles in Distal Segment** | 70-180 | 100-150 | 125 |
| **Length of Distal Bristle Segment (mm)** | 2-10 | 5-7 | 6.2 |
| **Membrane Diameter (mm)** | 11-20 | 13-15 | 14 |
| **Membrane thickness (µm)** | < 25 | < 18 | 8-16 |
| **Membrane Location** | Proximal and distal segment | Proximal and distal segment | Proximal segment |
| **Stem Wire Diameter (in)** | 0.004-0.010 | 0.005-0.008 | 0.006 |
| **Bristle Diameter (in)** | 0.001-0.0025 | 0.00175-0.002 | 0.02 |
| **Segment Diameter (mm)** | 14-38 | 18-30 | 25 |
| **Gap Between Segments** | 0.5-10 | 2-5 | 3-4 |
| **Direction of fibres in most proximal segment** | Proximally pointing | Proximally pointing | Proximally pointing |
| **Direction of fibres in most distal segment, or segments** | Distally pointing | Distally pointing | Distally pointing |

**Table 3b**

| **Attribute** | **0.054-0.060in ID Catheter** | | |
|---|---|---|---|
| | **Preferably 0.056in** | | |
| | **Implant for long vessel segment treatment** | | |
| | **Range** | **Preferably** | **More Preferably** |
| **Length of Implant (cm)** | 2-30 | 4-25 | 10-20 |
| **Suitable Vessel Diameter (mm)** | 2-20 | 3-15 | 3-12 |
| **Number of Bristle Segments** | 2-30 | 4-25 | 9-22, or approximately 1 segment per cm of implant length |
| **Number of Bristles in Proximal Segment** | 50-110 | 70-90 | 80 |
| **Length of Proximal Bristle Segment (mm)** | 2-6 | 2.8-4.2 | 3.5 |
| **Number of Bristles in Distal Segments** | 50-130 | 80-100 | 90 |
| **Length of Distal Bristle Segment (mm)** | 2.5-5 | 3.5-4.5 | 3.9 |
| **Membrane Location** | Proximal and distal segment | Proximal and distal segment | Proximal segment |
| **Membrane Diameter (mm)** | 11-20 | 13-15 | 14 |
| **Membrane Thickness (µm)** | < 25 | < 18 | 9-15 |
| **Stem Wire Diameter (in)** | 0.004-0.010 | 0.005-0.008 | 0.006-0.008 |
| **Bristle Diameter (in)** | 0.001-0.002 | - | 0.0175 |
| **Segment Diameter (mm)** | 14-38 | 18-30 | 25 |
| **Gap Between Segments (mm)** | 0.5-10 | 2-5 | 3-7 |
| **Direction of fibres in most proximal segment** | Proximally pointing | Proximally pointing | Proximally pointing |
| **Direction of fibres in most distal segment, or segments** | Distally pointing | Distally pointing | Distally pointing |

**Table 3c**

| **Attribute** | **Implant for long vessel segment treatment** | | |
|---|---|---|---|
| | | | |
| | **0.054-0.060in ID Catheter** | | |
| | **Preferably 0.056in** | | |
| | **Range** | **Preferably** | **More Preferably** |
| **Length of Implant (cm)** | 2-30 | 4-25 | 5-20 |
| **Suitable Vein Diameter (mm)** | 2-15 | 3-15 | 3-112 |
| **Suitable Artery Diameter (mm)** | 2-15 | 4-9 | 5-7 |
| **Number of Bristle Segments** | 2-30 | 4-25 | 9-22, or approximately 1 segment per cm of implant length |
| **Number of Bristles in Proximal Segment** | 50-110 | 70-90 | 80 |
| **Length of Proximal Bristle Segment (mm)** | 2-6 | 2.8-4.2 | 3.40 ± 0.5mm |
| **Number of Bristles in Distal Segments** | 50-130 | 80-100 | 90 |
| **Length of Distal Bristle Segment (mm)** | 2.5-5 | 3.5-4.5 | 3.70± 0.5mm |
| **Membrane Location** | Proximal and / or distal segment | Proximal and distal segment | Proximal segment |
| **Membrane Diameter (mm)** | 11-20 | 13-15 | 14 |
| **Membrane Thickness (µm)** | < 25 | < 18 | 10-16 |
| **Stem Wire Diameter (in)** | 0.004-0.010 | 0.005-0.008 | 0.006-0.008 |
| **Bristle Diameter (in)** | 0.001-0.002 | - | 0.0175 |
| **Segment Diameter (mm)** | 14-38 | 18-30 | 25 |
| **Gap Between Proximal Segments (mm)** | 0.5-10 | 2-7 | 3-4 |
| **Gap Between Distal Segments (mm)** | 0.5-10 | 2-7 | 6-6.5 |
| **Direction of fibres in most proximal segment** | Proximally pointing | Proximally pointing | Proximally pointing |
| **Direction of fibres in most distal segment, or segments** | Distally pointing | Distally pointing | Distally pointing |

In some instances, it is not possible to access a target vessel using a standard catheter or sheath (which usually has an inner diameter of 0.035-0.038 inches). For these instances a range of catheters have been developed known as microcatheters. These catheters exhibit excellent flexibility, and have an outer diameter typically less than 3.3 French. The internal diameter of these catheters ranges from 0.012 to 0.029 inches. Standard internal diameters are 0.021, 0.024 and 0.027 inches. For compatibility with such catheters devices of the invention with the following attributes are preferred.

**Table 4a**

| **Attribute** | | **0.021-0.029in ID Catheter** | |
|---|---|---|---|
| | | **Preferably 0.027in** | |
| | **Range** | **Preferably** | **More Preferably** |
| **Suitable Vessel Diameter (mm)** | 1.0-6.0 | - | 1.5 |
| **Bristle Diameter (in)** | 0.0005-0.002 | 0.0007-0.0015 | 0.001 |
| **Number of Segments** | 1-4 | 1-2 | 1 |
| **Number of Bristles in Segment** | 100-500 | 200-450 | 300-400 |
| **Length of Proximal Bristle Segment (mm)** | 2-10 | 3.5-9 | 7 |
| **Diameter of Proximal End of Segment (mm)** | 2-12 | 3-7 | 3 |
| **Diameter of Distal End of Segment (mm)** | 4-14 | 4-10 | 8 |
| **Stem Wire Diameter (in)** | 0.003-0.010 | 0.003-0.006 | 0.004-0.005 |
| **Stem Diameter (in)** | 0.05-0.020 | 0.005-0.015 | 0.007 |
| **Gap between segments (mm)** | None, or 1-5 | None, or 1-3 | Not Applicable |
| **Direction of fibres in most proximal segment** | Proximally pointing | Proximally pointing | Proximally pointing |
| **Direction of fibres in most distal segment, or segments** | Distally pointing | Distally pointing | Distally pointing |

**Table 4b**

| **Attribute** | **0.025-0.030 in ID Catheter** | | |
|---|---|---|---|
| | **Preferably ≥0.027in** | | |
| | **Implant for short vessel treatment** | | |
| | **Range** | **Preferably** | **More Preferably** |
| **Length of Implant (cm)** | 1.0-2.5 | ≤2.0 | ≤15 |
| **Suitable Artery Diameter (mm)** | 1.5-7 | 1.5-5 | 1.5-4.5 |
| **Number of Bristle Segments** | 1-3 | - | 2 |
| **Number of Bristles in Proximal Segment** | 100-200 | 115-135 | 125 |
| **Length of Proximal Bristle Segment (mm)** | 2-5 | 2.5-3.5 | 3 |
| **Number of Bristles in Distal Segments** | 50-120 | 70-90 | 80 |
| **Length of Distal Bristle Segment (mm)** | 2-5 | 2.5-3.5 | 3 |
| **Membrane Location** | Proximal and distal segment | Proximal and distal segment | Proximal segment |
| **Membrane Diameter (mm)** | 3-8 | 4-6 | 5 |
| **Membrane Thickness (µm)** | < 25 | < 18 | 9-15 |
| **Stem Wire Diameter (in)** | 0.002-0.006 | 0.003-0.004 | 0.004 |
| **Diameter of Bristle in Proximal Segment (in)** | 0.00075-0.002 | - | 0.001 |
| **Diameter of Bristle in Distal Segment (in)** | 0.001-0.002 | - | 0.0015 |
| **Segment Diameter (mm)** | 6-20 | -12 | 10 |
| **Gap Between Segments (mm)** | 0.5-4 | 1-2 | 1 |

**Table 4c**

| **Attribute** | **0.025-0.030 in ID Catheter** | | |
|---|---|---|---|
| | **Preferably 0.027in** | | |
| | **Implant for short vessel treatment** | | |
| | **Range** | **Preferably** | **More Preferably** |
| **Length of Implant (cm)** | 1.0-2.5 | ≤2.0 | ≤1.5 |
| **Suitable Artery Diameter (mm)** | 1.5-7 | 1.5-5 | 1.5-4.5 |
| **Number of Bristle Segments** | 1-3 | - | 2 |
| **Number of Bristles in Proximal Segment** | 40-100 | 40-75 | 40-60 |
| **Length of Proximal Bristle Segment (mm)** | 2-5 | 2.5-3.5 | 2 ± 0.5mm |
| **Number of Bristles in Distal Segments** | 40-80 | 40-60 | 40-60 |
| **Length of Distal Bristle Segment (mm)** | 2-5 | 2.5-3.5 | 2± 0.5mm |
| **Membrane Location** | Proximal and/or distal segment | Proximal and distal segment | Proximal segment |
| **Membrane Diameter (mm)** | 3-8 | 4-7 | 6 |
| **Membrane Thickness (µm)** | < 25 | < 18 | 7-13 |
| **Stem Wire Diameter (in)** | 0.001-0.005 | 0.004-0.004 | 0.003 |
| **Diameter of Bristle in Proximal Segment (in)** | 0.00075-0.002 | 0.001- 0.0015 | 0.0015 |
| **Diameter of Bristle in Distal Segment (in)** | 0.001-0.002 | 0.0010.0015 | 0.0015 |
| **Segment Diameter (mm)** | 6-20 | -12 | 10 |
| **Gap Between Segments (mm)** | 0.5-4 | 1-2 | 1 |

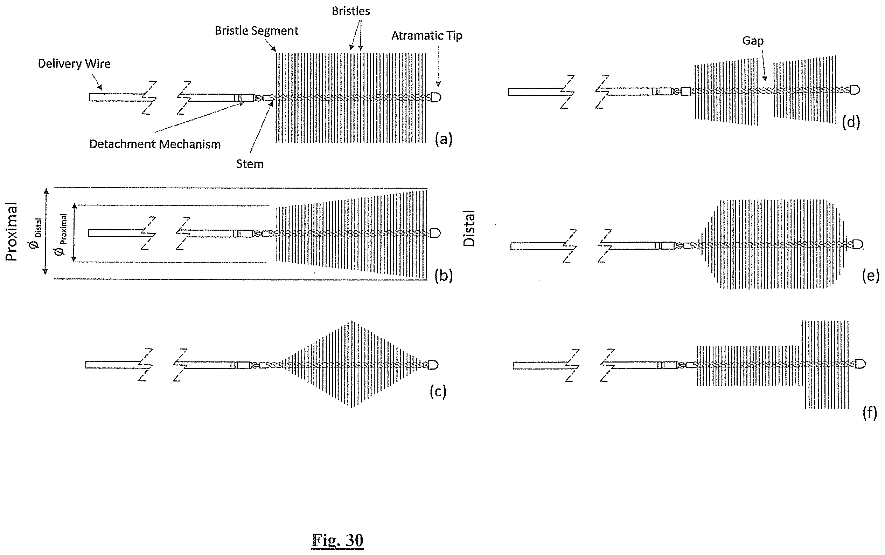

A range of geometries, incorporating gaps between segments may be used. In one embodiment a bristle segment of uniform diameter may be used (Fig. 30 (a)).

A lower profile collapsed configuration can be achieved for delivery through a microcatheter by using a taper in which the proximal diameter of the bristle segment is lower than the distal diameter of the bristle segment. This is shown schematically in Fig. 30 (b). This is achievable since the distal bristles do not need to collapse onto any stem distally, while the proximal bristles lie on all bristles which are present distally.

In order to ensure adequate anchoring of the implant in the vessel to prevent migration, a specific portion of the bristle segment may be designed such that a minimum degree of oversizing with respect to the vessel diameter is incorporated. The degree of taper introduced may be driven by this. In one configuration the lowest diameter of the bristle segment is at least that of the target vessel diameter. For example in Fig. 30 (f), the lower diameter proximal portion of the implant may be at least that of the target vessel, while the larger diameter may be substantially larger than the target vessel. In one embodiment the lower diameter portion of the segment may be 2-4 mm, while the larger diameter portion of the bristle segment may be 4-8 mm. In another embodiment the diameter of the bristle segment may be approximately the same as the target vessel.

In another configuration, a double tapered segment may be used (Fig. 30 (c)), or a number of individual tapered segments may be used (Fig. 30 (d)).

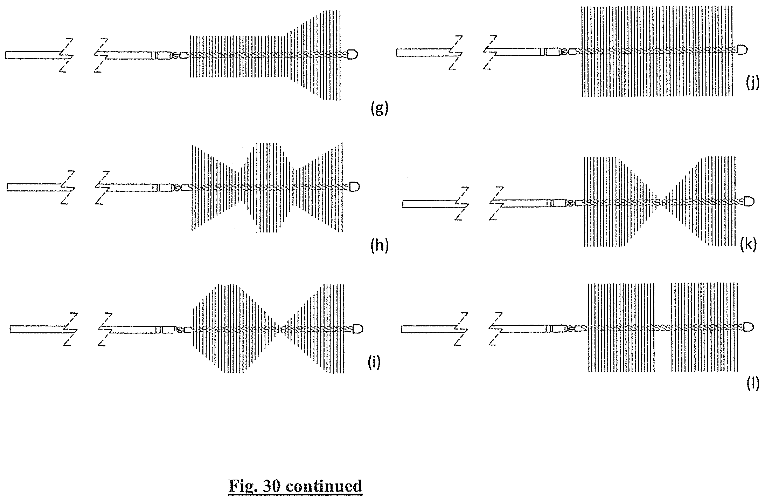

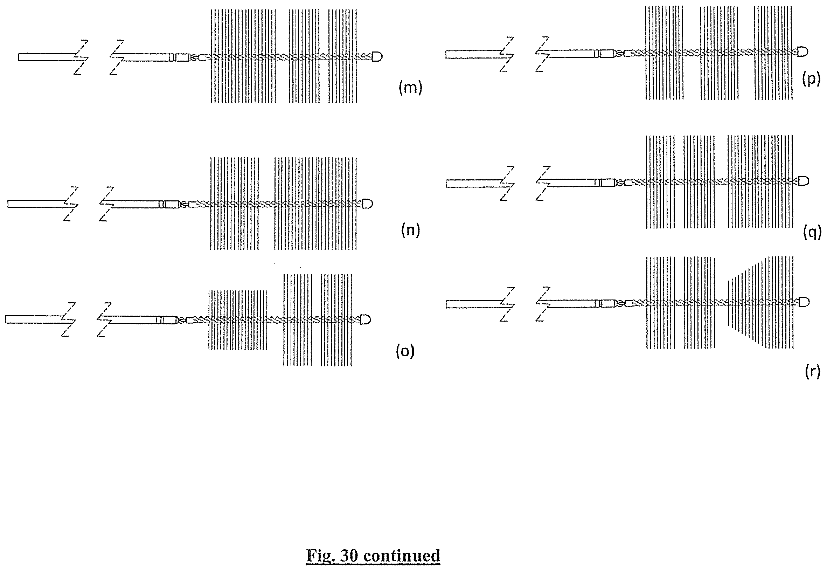

The use of a gap can further improve the efficiency (increase in the number of bristles) with which bristles can be placed within a catheter while maintaining a low profile implant in the collapsed condition. More fibres ensures better anchor force and increased interference with blood flow resulting in better thrombogenicity and shorter time to occlusion. Some examples are shown in Fig. 30 (m-r) and (d). Any combination of these features (gaps and tapers) can further increase the effectives of the implant in anchoring within the vessel and causing vessel occlusion.

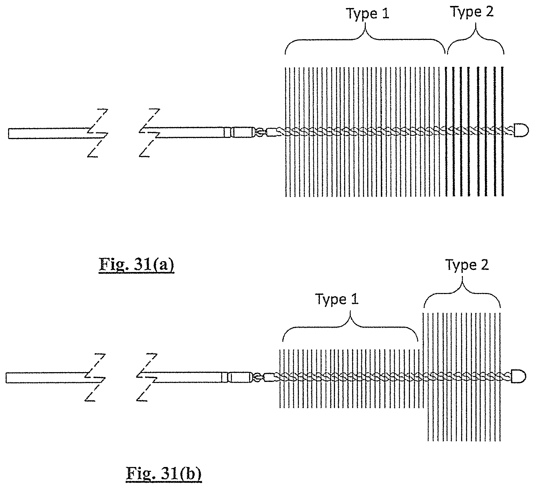

Another means to enable a profile sufficiently low to fit through a microcatheter is the use of bristles of differing types i.e. with different properties. For example, as illustrated in Fig. 31 a large number of fibres of a low diameter may be incorporated in one area of a segment to induce rapid thrombus formation and vessel occlusion. Similarly a lower number of fibres of a higher diameter may be incorporated. In one embodiment a group of fibres of diameter 0.0007 inches, and a group of fibres of 0.001 inches is used. In another instance a group of fibres of 0.001 and 0.0015 inches are used.

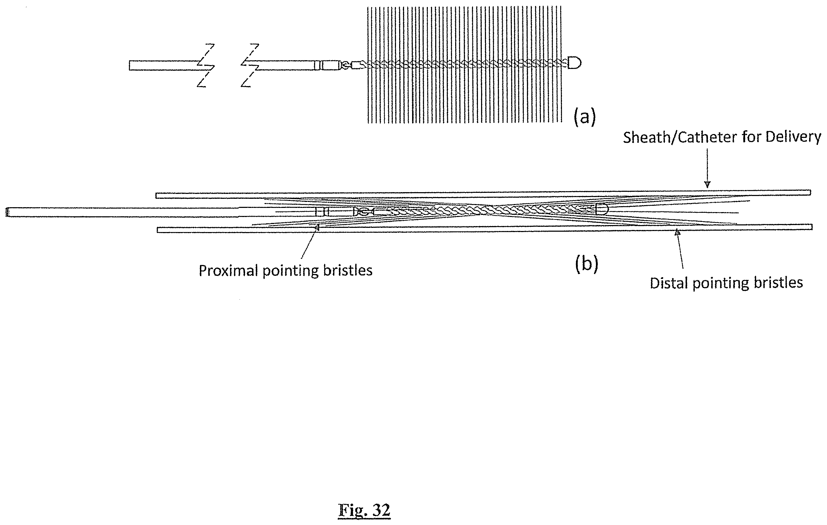

When the implant is placed into a catheter it is in a collapsed condition. If all bristles of a segment are collapsed such that they all point one direction, the bristles will lie on top of one another. This increases the profile of the segment in the collapsed condition. A longer segment with more bristles means a larger profile in the collapsed condition. One means to reduce the profile in the collapsed condition is to collapse some of the fibres such that they point distally, and others such that they point proximally. This is shown schematically in Fig. 32.

Following deployment the bristle segment may be resheathed. This will force all fibres which original pointed proximally to be flipped such that they point distally. In one embodiment there is sufficient space within the microcatheter to enable all bristles to enter the microcatheter. In another configuration a most distal portion of the segment may not fully enter the microcatheter due to insufficient space for the bristles i.e. the profile is too high when all fibres of the segment point distally.

In yet another embodiment, the amount and configurations of the bristle segment may be tuned such that while not all fibres can enter the catheter, due to insufficient space, the bristles which remain outside the catheter are aligned roughly parallel to the catheter centreline and thus do not contact the vessel wall. This ensures that if the physician wishes to remove the implant or alter its position he or she will cause damage or denudation of the vessel wall.

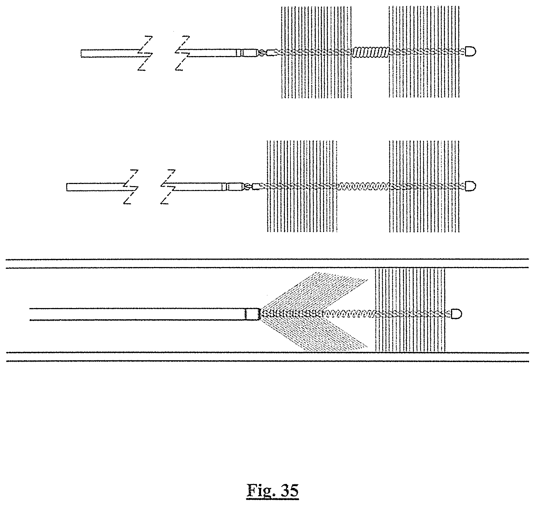

In yet another embodiment, an extendable connection exists between a distal and proximal segment. This will be advantageous particularly where the collapsed profile is too large to be resheathed due to proximal segment bristles overlapping the distal segment bristles. As the physician pulls the proximal segment into the catheter and as the distal segment begins to enter the catheter causing resistance the extendable connection will stretch increasing or enabling a gap to emerge between segments. The increase in the gap size can enable the collapse of more or all of the segments into the catheter. Fig. 35 (a) shows such a configuration with an extendable connection in the unloaded state. Fig. 35 (b) displays the same configuration in the loaded state with the extendable connection elongated. Fig. 35 (c) shows the resheathing step wherein the catheter collapses the proximal segment bristles. This action also causes an elongation of the extendable connection alleviating the degree of overlap of the proximal bristles onto the distal bristles. The extendable connection may comprise a spring or elastic element which can return to its original length upon unloading. The extendable connection may comprise non-elastic type element.

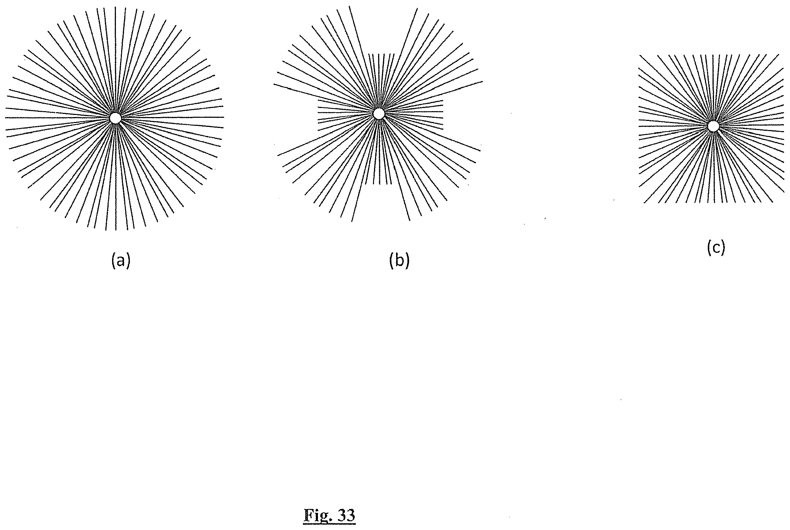

Another means to reduce the profile of the bristle segment is to trim the segment such that it has a non-circular cross section. This is shown schematically in Fig. 33. (a) shows a conventional bristle segment which has not been trimmed. Fig. 33 (b) shows a segment which has been trimmed such that there is a lower diameter region. In this way the longer fibres will serve to ensure the implant is well anchored in the vessel, while the shorter fibres will support thrombus formation. Other non-circular geometries such as a square Fig. 33 (c), triangular or others may be used.

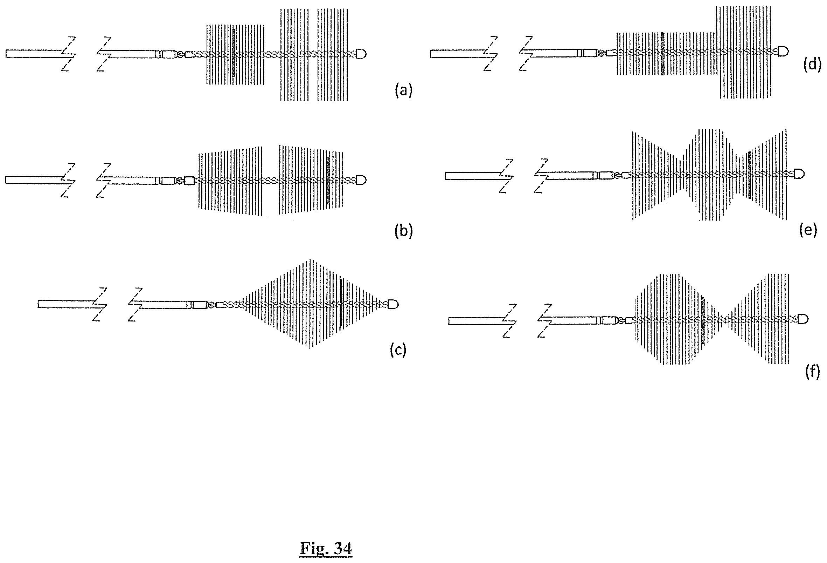

In some implants, a membrane or flow blocking member may be incorporated. A number of configurations are shown schematically in Fig. 34 which deal with the problem of space constraints within a microcatheter. Since the membrane will contribute significantly to the profile of the implant in the collapsed configuration, it may be advantageous to place the membrane in an area of the segment which is generally of low profile in the collapsed configuration. This low profile area of the segment may be achieved by any of the means described above (including reduced segment diameter, use of lower diameter bristles, use of tapers and the like).

As described previously, the implant may be detached via a screw mechanism. In one embodiment the female or male portion of the detachment mechanism is comprised of a radiopaque material. This is to facilitate visibility of detachment during use. In yet another embodiment both female and male portions of the screw detachment mechanism are radiopaque enabling the physician to distinctively see the male detach from the female.

In one embodiment a male portion of a screw detachment mechanism is attached to the implant, and the female to the delivery wire. In another embodiment the female portion of the screw detachment mechanism is attached to the implant and the male portion to the delivery wire.

A gap between segments as shown in Fig 9(a) can facilitate a low profile during delivery and retrieval. During retrieval, via re-sheathing of the implant into a catheter, the gap facilitates a low profile when the fibres of the proximal segment which may include a membrane are altered from a proximally pointing configuration to a distally pointing direction. In a situation wherein retrieval of the implant is not a desirable attribute the gap between the segments may be as low as 1 mm. In another embodiment there may be no gap between the distally and proximal segments.

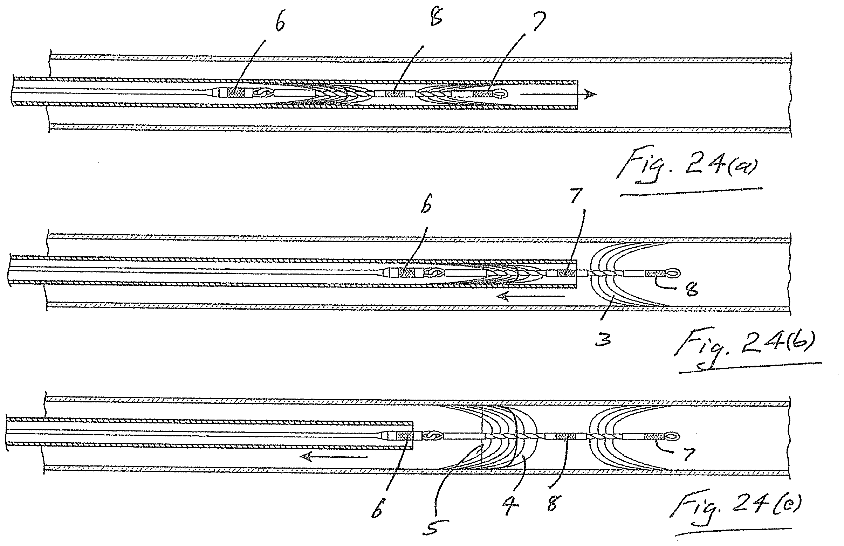

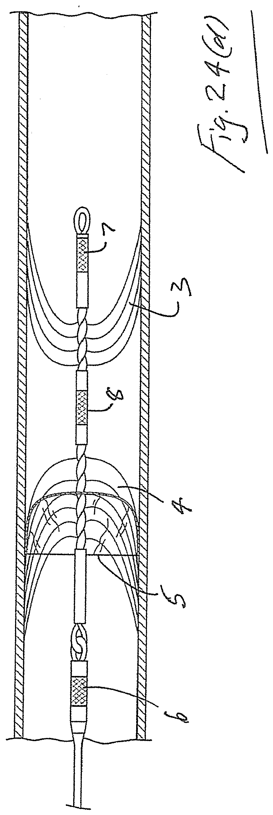

A further embodiment of the device, deliverable through a microcatheter, is described in Table 5. The design is similar to that shown in Fig. 24. In one configuration a larger bristle diameter is used in the distal segment than the proximal segment. This is to ensure maximum outward radial force from the distal segment helping to anchor the device. A lower bristle diameter may be utilised in the proximal segment in order to facilitate a membrane in the proximal segment while also being deliverable through a microcatheter.

As discussed previously, the oversizing of the device diameter compared to the vessel (calculated as the percentage difference in diameter between segment diameter and the vessel diameter) is preferably at least 20%, more preferably 50% of the vessel diameter and more preferably at least 100% of the vessel diameter in which the device is implanted. Even more preferably, 150% oversizing should be employed.

### Embolization Procedures

Embolization procedures may be undertaken by a range of physicians, primarily interventional radiologists, endovascular surgeons, and interventional cardiologists. There are a number of indications for embolization. Frequently performed procedures, and the associated physician are summarised in the table below.

In embolization in general the flow direction is from proximal to the distal (prograde, or away from the heart). This is the natural flow direction in arteries. In healthy veins, the flow direction will generally be the opposite (retrograde, towards the heart). However in general, embolization is performed in patients with reflux meaning flow will also be prograde.

Because the flow will generally be from proximal to distal it is preferable to have a membrane on the proximal end of the device open proximally, with the bristles also pointing proximally. This mitigates any potential for flow to pass around the outside of the membrane.

| **Indication** | **Primary Physician** | **Target Vessel for Occlusion** | **Access Site** | **Direction of Flow** | **Notes** |
|---|---|---|---|---|---|
| **Assisted Maturation of Dialysis Access Fistulas** | Endovascular Surgeon, Interventional Radiologist | Accessory Veins in AVF | Radial Artery | Prograde | The accessory veins are draining the venous outflow, therefore flow is the opposite of a normal accessory vein. Catheter tip points in direction of flow during device delivery. |
| | | Accessory Veins in AVF | Venous Outflow | Prograde | The accessory veins are draining the venous outflow, therefore flow is the opposite of a normal accessory vein. Catheter tip points in direction of flow during device delivery. |
| **Hemoptysis** | Interventional Radiologist | Bronchial Artery | Femoral / Radial Artery | Prograde | Catheter tip points in direction of flow during device delivery |
| **Pre Op Y-90 (prevent non-target embolization)** | Interventional Radiologist | Gastroduod enal, Gastric, Cystic Artery | Femoral / Radial Artery | Prograde | Catheter tip points in direction of flow during device delivery |
| **Varicocele** | Vascular Surgeon, Interventional Radiologist | Gonadal/ Ovarian Vein | Jugular Vein / Femoral | Prograde | Refluxing/diseased vessel, so flow is away from heart. Catheter tip points in direction of flow during device delivery. Membrane may be used to control sclerosant to treat collateral vessels. |
| **Liver Metastases** | Interventional Radiologist | Hepatic Artery | Femoral/Radial Artery | Prograde | Catheter tip points in direction of flow during device delivery |
| **Type II Endoleaks** | Vascular Surgeon | Inferior Mesenteric Artery, Internal Iliac Arteries | Femoral/Radial Artery | Prograde | Catheter tip points in direction of flow during device delivery |
| **Pelvic Congestion Syndrome** | Vascular Surgeon, Interventional Radiologist | Ovarian Vein, Internal or Pudendal Vein | Jugular Vein/Femoral | Prograde | Refluxing/diseased vessel, so flow is away from heart. Catheter tip points in direction of flow during device delivery. Membrane may be used to control sclerosant to treat collateral vessels. |
| **Hemorrhoids** | Vascular Surgeon, Interventional Radiologist | Internal Iliac or Pudendal Vein | Jugular Vein/Femoral | Prograde | Refluxing/diseased vessel meaning haemorrhoidal plexus is not draining properly. Flow is away from heart into the plexus. Catheter tip points in direction of flow during device delivery |
| | | Hemorroida 1 Arteries | Femoral/Radial Artery | Prograde | Catheter tip points in direction of flow during device delivery |
| **Liver Cancer: Promotion of future remnant hypertrophy** | Interventional Radiologist | Portal Vein | Trans-hepatic entry via contralateral approach | Prograde | Catheter tip points in direction of flow during device delivery |
| | Interventional Radiologist | Portal Vein | Trans-hepatic entry via ipsilateral approach | Prograde | Depending on orientation of catheter/tip, could be retrograde flow. |
| | Interventional Radiologist | Portal Vein | Jugular Vein | Prograde | Catheter tip points in direction of flow during device delivery |
| **Aneurysms** | Interventional Radiologist | Splenic, hepatic artery | Femoral or Radial Artery | Prograde | Catheter tip points in direction of flow during device delivery |
| **Haemorrhage** | Vascular Surgeon, Interventional Radiologist | Any artery | Radial or Femoral Artery | Prograde | Catheter tip points in direction of flow during device delivery |

### Treatment of an aneurysm with a membrane at distal and proximal ends

Normally blood flows from proximal to distal in the parent vessel, past an aneurysm, with some filling of the aneurysm sac. It may therefore seem intuitive that occlusion of the proximal inflow towards the aneurysm should prevent flow into the sac. However, in some scenarios occlusion of the proximal vessel can alter the hemodynamics of the vessels locally, meaning flow can travel from distal to the aneurysm causing further filling and pressurising of the aneurysm sac. In this scenario the physician aims to occlude the parent vessel proximal and distal to the aneurysm. This is known as front-door backdoor treatment of the aneurysm.

In one embodiment the device may be configured such that there is both a proximal and distal membrane on the device, enabling rapid occlusion of the parent vessel both proximal and distal to the aneurysm. Accordingly the proximal membrane is configured to be proximal the aneurysm sac, while the distal membrane is distal to the membrane sac. In a preferable configuration the membrane on the proximal bristle segment is open proximally, and the membrane on the distal segment is open distally.

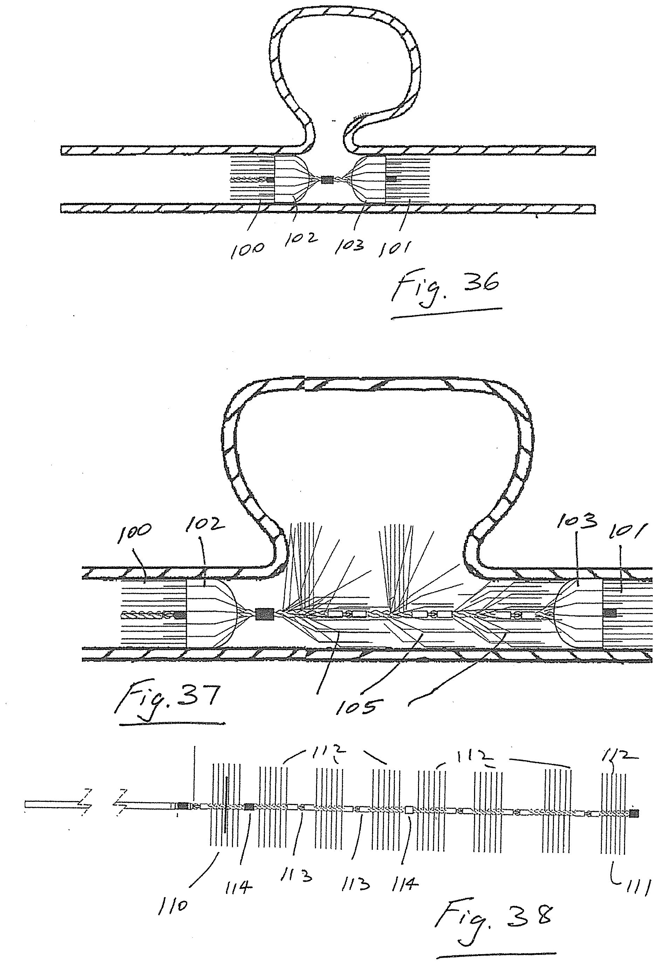

One arrangement with two bristle segments 100, 101 each containing a membrane 102, 103 is illustrated in Fig. 36. Another arrangement with several additional segments 105 to bridge a larger aneurysm is illustrated in Fig. 37.

### Use of a Stiff and Flexible Interconnects Between Distal Segments in Longer Device

In some instances the properties of the segments may be such that no flexible connections in required. For short devices flexible connections may not be required. However for longer devices some flexibility may be required. It is preferable that at least one flexible connection per 5cm of the implant length be present.

In one configuration, a device has many distal bristle segments in which the distal segments are connected via both flexible and stiff connections. This may be required when flexible connections between all distal segments mean that the pushability of the implant when being delivered through a catheter is compromised due to too much flexibility. This may be the case in particular where very flexible connections incorporating a hinge are used. The replacement of at least one flexible connection with a stiff or stiffer connection will improve the column stiffness of the implant and hence its pushability. This will reduce the force required to push the implant through the delivery catheter. It may be preferable to place the stiff connections intermittently between the flexible sections to ensure good flexibility along the length while also maintaining good pushability along the length.

One such device is illustrated in Fig. 38. This device has proximal and distal segment 110, 111 and a plurality of intermediate segments 112. Some of the connections between the segments are hinged 113 and others are relatively stiff 114.

It is preferable that the membrane is within the bristle segment. A bristle manipulating tool may be used and some of the bristles may be manipulated so that the bristles are aligned with the stem. A flow restrictor membrane is mounted between the bristles and thereafter the bristles are released from the tool or vice versa.

When the bristles recover the membrane will be between and protected and secured by bristles both proximally and distally.

To ensure that the membrane is controlled by the adjacent bristles, some bristles should be present both distal and proximal to the membrane. In one confirmation the membrane 130 is placed such that 50% of bristles are proximal to the membrane while 50% are distal to the membrane. To prevent overlap of the membrane onto structures proximal to the segment (such as a detach mechanism, or delivery wire), the membrane may be placed more distally within the segment. In one configuration 60% of fibres are proximal to the membrane while 40% are distal to the membrane. In another configuration 70% of fibres are proximal to the membrane while 30% are distal to the membrane. In another configuration 80% of fibres are proximal to the membrane while 20% are distal to the membrane.

### Membrane Hole Diameter and Interference Fit

Placement of the membrane within the bristle segments ensures that bristles inhibit the membrane from translating proximally or distally along the segment while in use or when deployed. The security of the membrane may be further improved via an interference fit between a hole in the membrane and of the segment stem. To achieve this, the hole in the membrane should be smaller than the stem of the segment. Once placed onto the stem the mismatch of the diameter of the stem and the hole in the membrane will cause friction between the two surfaces and an interference fit.

To achieve an interference fit the hole in the membrane should be less the stem diameter. Preferably the hole diameter in the membrane should be at least 0.001in less than the diameter of the stem. More preferably the hole diameter in the membrane should be at least 0.002in less than the diameter of the stem.

It will be appreciated that if the hole in the membrane is too small, excessive stretching may be required to apply the membrane to the segment causing irrecoverable deformation of the hole such that no interference may be present or a gap could exist between the stem and the hole in the membrane. In this instance some flow may pass through this gap inhibiting the device performance in terms of occlusion. The hole of the membrane should be no more than 40% less than the diameter of the stem.

Ideally the hole diameter is specified such that the ratio between the initial hole diameter and stretched hole diameter should be less than the ultimate elongation of the membrane material such that the membrane hole diameter will recover to its lower diameter causing interference fit with the stem.

The device disclosed may also be used in fields beyond embolization. For example, these embodiments may be particularly useful in the field of contraception wherein the fallopian tubes are occluded. Furthermore the device disclosed may be used in the field of bronchiopulmonary occlusion. For example, in the case where a physician wishes to exclude a portion of the lung by occluding a bronchus.

Modifications and additions can be made to the embodiments of the invention described herein without departing from the scope of the invention. For example, while the embodiments described herein refer to particular features, the invention includes embodiments having different combinations of features. The invention also includes embodiments that do not include all of the specific features described, as long as these embodiments fall within the appended claims.

The invention is not limited to the embodiments hereinbefore described, with reference to the accompanying drawings, which may be varied in construction and detail.

### References

1. Ekeh et al., Complications arising from splenic artery embolisation: a review of an 11-year experience. The American Journal of Surgery, 205, 250-254, 2013
2. Ryer et al. 2013, Comparison of outcomes with coils versus vascular plug embolisation of the internal iliac artery for endovascular aortoiliac aneurysm repair. Journal of Vascular Surgery, Volume 56, Issue 5, November 2012, Pages 1239-1245.
3. Beddy et al., Testicular varicoceles. Clinical Radiology (2005) 60, 1248-1255
4. Beecroft etal., Percutaneous varicocele embolisation. Canadian Urological Association Journal. September 2007, Volume 1, Issue 3
5. Kessel et al., Transcatheter Embolisation and Therapy. Springer ISBN 978-1-84800-896-0. Published 2010
6. Balian et al. Pelviperineal venous insufficiency and varicose veins of the lower limbs. Phlebolymphology. 2008; 15(1):17-26.
7. Marsh et al., Coil Protruding into the Common Femoral Vein Following Pelvic Venous Embolisation. Cardiovascular Interventional Radiology (2008) 31:435-438
8. The Technology of Expansion. Terumo Interventional Systems. Downloaded on February 21, 2013 from http://www.terumois.com/products/embolics/AZUR.aspx
9. Letourneau-Guillon et al., Embolisation of Pulmonary Arteriovenous Malformations with Amplatzer Vascular Plugs: Safety and Midterm Effectiveness. Journal of Vascular and Interventional Radiology, Volume 21, Issue 5, Pages 649-656, May 2010.
10. Yoo et al., Preoperative portal vein embolisation using an amplatzer vascular plug. European Radiology (2009) 19: 1054-1061.
11. Pelage et al. What is Azur Hydrocoil and How Does it Work? Presented at Society of Interventional Radiology, 2011.

## Claims

1. An embolization device, comprising:
a proximal segment (4);
a distal segment (3), each of the proximal and distal segments including
a stem (20), and
a plurality of anchoring bristles extending outwardly from the stem; and
a flow restricting membrane (5) located longitudinally within the bristles of one of said segments, and wherein the flow restricting membrane has a central hole, **characterized in that** there is an interference fit between the central hole and the stem.

2. A device as claimed in claim 1, wherein the flow restricting membrane is disposed in a low profile area of one of the at least two segments.

3. A device as claimed in claim 1 or 2 wherein the flow restricting membrane is located adjacent to a proximal end of the device, and, optionally, wherein the flow restricting membrane is located within or adjacent to the most proximal segment (4).

4. A device as claimed in any of claims 1 to 3 wherein the flow restricting membrane is located adjacent to the distal end of the device, and, optionally, wherein the flow restricting membrane is located within or adjacent to the most distal segment.

5. A device as claimed in any preceding claim:
wherein the bristles in an unconstrained configuration extend to a radial extent which is greater than the radial extent of the flow restricting membrane in the unconstrained configuration; and/or
wherein, in the constrained configuration, the flow restricting membrane has a longitudinal extent, and, optionally, wherein, in the deployed configuration, the flow restricting membrane has a conical or cup-like shape.

6. A device as claimed in any of claims 1 to 5 wherein the flow restricting membrane is of a flexible material, and, optionally, wherein the flow restricting membrane is of a polymeric material and/or an elastomeric material.

7. A device as claimed in any of claims 1 to 6 wherein the flow restricting membrane comprises a film and/or a shape memory material such as Nitinol.

8. A device as claimed in any of claims 1 to 7, wherein the distal segment (13) includes more bristles than the proximal segment (4).

9. A device as claimed in any of claims 1 to 8, wherein the flow restricting membrane is located on the proximal segment, and wherein a connection between the two most proximal segments is stiff.

10. A device as claimed in any preceding claim, wherein the flow restricting membrane does not overlap with the detachment mechanism (41) when the flow restricting membrane is collapsed proximally.

11. A device as claimed in any preceding claim, wherein the central hole is smaller than the stem and/or wherein the central hole has a diameter which is smaller than the diameter of the stem.

12. An embolization system, comprising a delivery catheter and an embolization device as claimed in any of claims 1 to 11 loaded in the delivery catheter (10).

13. A method for manufacturing an embolization device comprising the steps of:-
providing a bristle segment (3, 4) having a plurality of bristles extending outwardly of the stem (20);
providing a bristle manipulating tool;
manipulating at least some of the bristles so that the bristles are aligned with the stem;
mounting a flow restrictor membrane (5) between the bristles; and
releasing the bristles from the tool,
wherein the method comprises the step of mounting the membrane on the stem of the bristle segment, wherein the membrane comprises a central hole and the method comprises engaging the stem in the hole of the membrane, and, wherein there is an interference fit between the central hole and the stem.

14. A method as claimed in claim 13 wherein the central hole adapts its shape and dimension at least in part to the shape and dimensions of a cross section of the stem.

15. A method as claimed in claims 13 or 14 wherein the central hole is stretched during mounting in order to fit the stem.

## Patentansprüche

1. Embolisationsvorrichtung, umfassend:
ein proximales Segment (4);
ein distales Segment (3), wobei jedes des proximalen und distalen Segments beinhaltend
einen Stiel (20) und
eine Vielzahl von Verankerungsborsten, die sich von dem Stiel nach außen erstrecken; und
eine durchflussbeschränkende Membran (5), die sich längsgerichtet innerhalb der Borsten von einem der Segmente befindet, und
wobei die durchflussbeschränkende Membran ein zentrales Loch aufweist, **dadurch gekennzeichnet, dass** es zwischen dem zentralen Loch und dem Stiel eine Übermaßpassung gibt.

2. Vorrichtung nach Anspruch 1, wobei die durchflussbeschränkende Membran in einem Niedrigprofilbereich von einem der mindestens zwei Segmente angeordnet ist.

3. Vorrichtung nach Anspruch 1 oder 2, wobei die durchflussbeschränkende Membran sich angrenzend an ein proximales Ende der Vorrichtung befindet und, wahlweise wobei die durchflussbeschränkende Membran sich innerhalb oder angrenzend an das proximalste Segment (4) befindet.

4. Vorrichtung nach Anspruch 1 bis 3, wobei die durchflussbeschränkende Membran sich angrenzend an das distale Ende der Vorrichtung befindet und, wahlweise, wobei die durchflussbeschränkende Membran sich innerhalb oder angrenzend an das distalste Segment befindet.

5. Vorrichtung nach einem der vorstehenden Ansprüche:
wobei die Borsten sich in einer uneingeschränkten Konfiguration in eine radiale Ausdehnung erstrecken, die größer ist als die radiale Ausdehnung der durchflussbeschränkenden Membran in der uneingeschränkten Konfiguration; und/oder
wobei die durchflussbeschränkende Membran in der eingeschränkten Konfiguration eine längsgerichtete Ausdehnung aufweist, und wahlweise wobei in der eingesetzten Konfiguration die durchflussbeschränkende Membran eine konische oder tassenartige Form aufweist.

6. Vorrichtung nach einem der Ansprüche 1 bis 5, wobei die durchflussbeschränkende Membran aus einem flexiblen Material besteht, und wahlweise wobei die durchflussbeschränkende Membran aus einem Polymermaterial und/oder einem Elastomermaterial besteht.

7. Vorrichtung nach einem der Ansprüche 1 bis 6, wobei die durchflussbeschränkende Membran eine Folie und/oder ein Formgedächtnismaterial wie Nitinol umfasst.

8. Vorrichtung nach einem der Ansprüche 1 bis 7, wobei das distale Segment (13) mehr Borsten als das proximale Segment (4) beinhaltet.

9. Vorrichtung nach einem der Ansprüche 1 bis 8, wobei die durchflussbeschränkende Membran sich an dem proximalen Segment befindet, und wobei eine Verbindung zwischen den zwei proximalsten Segmenten starr ist.

10. Vorrichtung nach einem vorstehenden Anspruch, wobei die durchflussbeschränkende Membran nicht mit dem Ablösemechanismus (41) überlappt, wenn die durchflussbeschränkende Membran proximal kollabiert wird.

11. Vorrichtung nach einem der vorstehenden Ansprüche, wobei das zentrale Loch kleiner ist als der Stiel und/oder wobei das zentrale Loch einen Durchmesser aufweist, der kleiner ist als der Durchmesser des Stiels.

12. Embolisationssystem, umfassend einen Einführkatheter und eine in dem Einführkatheter (10) geladene Embolisationsvorrichtung nach einem der Ansprüche 1 bis 11.

13. Verfahren zum Herstellen einer Embolisationsvorrichtung, umfassend die Schritte des:
Bereitstellens eines Borstensegments (3, 4) mit einer Vielzahl von Borsten, die sich von dem Stiel (20) nach außen erstrecken;
Bereitstellens eines borstenmanipulierenden Werkzeugs;
Manipulierens mindestens einiger der Borsten, sodass die Borsten mit dem Stiel ausgerichtet sind;
Montierens einer durchflussbeschränkenden Membran (5) zwischen den Borsten; und
Freigebens der Borsten von dem Werkzeug,
wobei das Verfahren den Schritt des Montierens der Membran an dem Stiel des Borstensegments umfasst, wobei die Membran ein zentrales Loch umfasst und das Verfahren das Eingreifen des Stiels in das Loch der Membran umfasst, und wobei es eine Übermaßpassung zwischen dem zentralen Loch und dem Stiel gibt.

14. Verfahren nach Anspruch 13, wobei das zentrale Loch seine Form und Abmessung mindestens zum Teil der Form und Abmessung eines Querschnitts des Stiels anpasst.

15. Verfahren nach Ansprüchen 13 oder 14, wobei das zentrale Loch während des Montierens gedehnt wird, um dem Stiel zu passen.

## Revendications

1. Dispositif d'embolisation, comprenant :
un segment proximal (4) ;
un segment distal (3), chacun des segments proximal et distal incluant une tige (20), et
une pluralité de brins d'ancrage s'étendant vers l'extérieur de la tige ; et
une membrane limitant l'écoulement (5) située de manière longitudinale dans les brins de l'un desdits segments, et
dans lequel la membrane limitant l'écoulement présente un orifice central, **caractérisé en ce qu'**il existe un ajustement des interférences entre l'orifice central et la tige.

2. Dispositif selon la revendication 1, dans lequel la membrane limitant l'écoulement est agencée dans une zone de profil bas de l'un des au moins deux segments.

3. Dispositif selon la revendication 1 ou 2, dans lequel la membrane limitant l'écoulement est située de manière adjacente à une extrémité proximale du dispositif, et, facultativement, dans lequel la membrane limitant l'écoulement est située dans ou de manière adjacente au segment le plus proximal (4).

4. Dispositif selon l'une quelconque des revendications 1 à 3, dans lequel la membrane limitant l'écoulement est située de manière adjacente à l'extrémité distale du dispositif, et, facultativement, dans lequel la membrane limitant l'écoulement est située dans ou de manière adjacente au segment le plus distal.

5. Dispositif selon l'une quelconque des revendications précédentes :
dans lequel les brins selon une configuration non contrainte s'étendent vers une extension radiale qui est supérieure à l'extension radiale de la membrane limitant l'écoulement selon la configuration non contrainte ; et/ou
dans lequel, selon la configuration contrainte, la membrane limitant l'écoulement présente une extension longitudinale, et, facultativement, dans lequel, selon la configuration déployée, la membrane limitant l'écoulement présente une forme conique ou de coupelle.

6. Dispositif selon l'une quelconque des revendications 1 à 5, dans lequel la membrane limitant l'écoulement est fabriquée à partir d'un matériau souple, et, facultativement, dans lequel la membrane limitant l'écoulement est fabriquée à partir d'un matériau polymère et/ou d'un matériau élastomère.

7. Dispositif selon l'une quelconque des revendications 1 à 6, dans lequel la membrane limitant l'écoulement comprend un matériau à mémoire de film et/ou de forme tel que le nitinol.

8. Dispositif selon l'une quelconque des revendications 1 à 7, dans lequel le segment distal (13) inclut plus de brins que le segment proximal (4).

9. Dispositif selon l'une quelconque des revendications 1 à 8, dans lequel la membrane limitant l'écoulement est située sur le segment proximal, et dans lequel une liaison entre les deux segments les plus proximaux est rigide.

10. Dispositif selon l'une quelconque des revendications précédentes, dans lequel la membrane limitant l'écoulement ne chevauche pas le mécanisme de libération (41) lorsque la membrane limitant l'écoulement est repliée de manière proximale.

11. Dispositif selon l'une quelconque des revendications précédentes, dans lequel l'orifice central est plus petit que la tige et/ou dans lequel l'orifice central présente un diamètre qui est inférieur au diamètre de la tige.

12. Système d'embolisation, comprenant un cathéter d'administration et un dispositif d'embolisation selon l'une quelconque des revendications 1 à 11 chargé dans le cathéter d'administration (10).

13. Procédé de fabrication d'un dispositif d'embolisation comprenant les étapes de :
fourniture d'un segment de brins (3, 4) présentant une pluralité de brins s'étendant vers l'extérieur de la tige (20) ;
fourniture d'un outil de manipulation des brins ;
manipulation d'au moins certains des brins de sorte que les brins soient alignés avec la tige ;
montage d'une membrane réductrice de débit (5) entre les brins ; et
libération des brins de l'outil,
dans lequel le procédé comprend l'étape de montage de la membrane sur la tige du segment de brins, dans lequel la membrane comprend un orifice central et le procédé comprend la mise en prise de la tige dans l'orifice de la membrane, et, dans lequel il existe un ajustement des interférences entre l'orifice central et la tige.

14. Procédé selon la revendication 13, dans lequel l'orifice central adapte sa forme et sa dimension au moins en partie à la forme et aux dimensions d'une section transversale de la tige.

15. Procédé selon les revendications 13 ou 14, dans lequel l'orifice central est étiré pendant le montage afin qu'il s'ajuste à la tige.
